# EUROPEAN PATENT SPECIFICATION

(11) **EP 3 495 350 B1**
(45) Date of publication and mention of the grant of the patent: **20.10.2021**
(21) Application number: 17834751.4
(22) Date of filing: 25.07.2017
(51) Int. Cl.: C07D 215/36, A61K 31/47, A61P 35/00

(54) **COMPOUND INHIBITING FORMATION OF C-MYC/MAX/DNA COMPLEX**
VERBINDUNG ZUR HEMMUNG DER BILDUNG EINES C-MYC/MAX/DNA-KOMPLEXES
COMPOSÉ INHIBANT LA FORMATION DU COMPLEXE C-MYC/MAX/ADN

(30) Priority: 29.07.2016 KR 20160097429; 24.07.2017 KR 20170093661
(43) Date of publication of application: 12.06.2019
(73) Proprietor: National Cancer Center, Goyang-si, Gyeonggi-do 10408 (KR); Korea Research Institute of Chemical Technology, Yuseong-gu, Daejeon 34114 (KR)
(72) Inventor: JEONG, Kyung Chae, Gyeonggi-do 10306 (KR); LIM, Hwan Jung, Daejeon 34049 (KR); PARK, Seong Jun, Daejeon 34049 (KR); SEO, Ho Kyung, Gyeonggi-do 10884 (KR); AHN, Kyung Ohk, Gyeonggi-do 15875 (KR); LEE, Sang Jin, Gyeonggi-do 10907 (KR); LEE, Eun Sook, Gyeonggi-do 13836 (KR)
(74) Representative: Dehns
(86) International application number: PCT/KR2017/008020
(87) International publication number: WO 2018/021810

(56) References cited:
- WO-A1-2008/102932
- JONG HEE CHOI ET AL: "Isothiazole Ring Formation with Substituted 2-Alkylthio-3-acyl-4-quinolinone Using O- (Mesitylenesulfonyl)hydroxylamine (MSH)", SYNLETT, no. 2, 1 January 2003 (2003-01-01), pages 0166-0172, XP055459586, DE ISSN: 0936-5214, DOI: 10.1055/s-2003-36802
- EBRAHEEM M A ET AL: "Synthesis of new polysubstituted (pyrazoles, pyrimidines and quinolines) five and six-membered heterocycles: reaction of @a,@a-dioxoketene dithioacetals with nucleophiles", TETRAHEDRON LETTERS, ELSEVIER LTD, AMSTERDAM, NL, vol. 51, no. 27, 7 July 2010 (2010-07-07), pages 3486-3492, XP027067185, ISSN: 0040-4039, DOI: 10.1016/J.TETLET.2010.04.003 [retrieved on 2010-06-01]
- HWANG B H ET AL: "A methodology for the synthesis of highly functionalized 2- and 4-aminoquinoline derivatives", TETRAHEDRON, ELSEVIER SCIENCE PUBLISHERS, AMSTERDAM, NL, vol. 64, no. 28, 7 July 2008 (2008-07-07), pages 6698-6704, XP022732000, ISSN: 0040-4020, DOI: 10.1016/J.TET.2008.05.014 [retrieved on 2008-05-09]
- SUK-KYEONG JUNG ET AL: "Identification of 3-acyl-2-phenylamino-1,4-dihydroquinolin-4 -one derivatives as inhibitors of the phosphatase SerB653 in, implicated in periodontitis", BIOORGANIC & MEDICINAL CHEMISTRY LETTERS, PERGAMON, AMSTERDAM, NL, vol. 22, no. 5, 4 January 2012 (2012-01-04), pages 2084-2088, XP028459460, ISSN: 0960-894X, DOI: 10.1016/J.BMCL.2012.01.011 [retrieved on 2012-01-21] & Suk-Kyeong Jung: "Supplementary data Synthesis of 2-amino-4-quinolinone", , 1 March 2012 (2012-03-01), XP055645975, Retrieved from the Internet: URL:https://ars.els-cdn.com/content/image/ 1-s2.0-S0960894X12000248-mmc1.doc [retrieved on 2019-11-25]
- JEONG KYUNG-CHAE ET AL: "Intravesical Instillation of c-MYC Inhibitor KSI-3716 Suppresses Orthotopic Bladder Tumor Growth", JOURNAL OF UROLOGY, LIPPINCOTT WILLIAMS & WILKINS, BALTIMORE, MD, US, vol. 191, no. 2, 17 July 2013 (2013-07-17) , pages 510-518, XP028813254, ISSN: 0022-5347, DOI: 10.1016/J.JURO.2013.07.019
- DATABASE Registry CAS; 9 August 2006 (2006-08-09), retrieved from STN Database accession no. RN 899793-55-6
- EBRAHEEM, M. A. et al.: "Synthesis of New Polysubstituted (pyrazoles, pyrimidines and quinolines) Five and Six-membered Heterocycles: Reaction of a, a-dioxoketene Dithioacetals with Nucleophiles", Tetrahedron Letters, vol. 51, no. 27, 2010, pages 3486-3492, XP027067185, DOI: doi:10.1016/j.tetlet.2010.04.003
- DATABASE Regsitry CAS; 7 January 2011 (2011-01-07), retrieved from STN Database accession no. RN 1258711-66-8
- CHOI, J. H. et al.: "Isothiazole Ring Formation with Substituted 2-alkylthio- 3-acyl-4-quinolinone Using O-(mesitylenesulfonyl)hydroxylamine (MSH", Synlett, vol. 2, 2003, pages 166-172, XP055459586, DOI: doi:10.1055/s-2003-36802
- DATABASE Registry CAS; 13 July 2017 (2017-07-13), retrieved from STN Database accession no. RN 2101913-76-0

## Description

### [Technical Field]

The present invention relates to a new class of compounds having c-Myc / Max / DNA complex formation inhibitory activity.

### [Background Art]

*c-myc* is a proto-oncogene encoding the c-Myc oncoprotein regulating cell transformation, growth, and differentiation, apoptosis, cell cycle progression and the like. Myc family proteins including c-Myc form a heterodimer with the basic/helix-loop-helix/leucine zipper (bHLHZip) domain of Max protein, and the Myc/Max heterodimer binds to a specific DNA sequence (CACGTG, i.e., E-box motif). Heterodimer formation with Max protein and subsequent DNA binding of the heterodimer are steps required for transcriptional activation of c-Myc target genes, and play important roles in promoting cell proliferation, malignant transformation, apoptosis and the like (see International Patent Publication No. WO2015/089180).

Abnormal expression of *c-myc* has been reported to be associated with a variety of cancers, including lung cancer, colorectal cancer, colon cancer, rectal cancer, breast cancer, bladder cancer, leukemia, myelogenous leukemia, lymphoma, small cell lung cancer, lung cancer, cervical carcinoma, osteosarcoma, glioblastoma, melanoma and the like (see Nature 1983 Nov 10-16; 306(5939): 194-196; Cancer Res 1985 Apr; 45(4): 1823-1827; and Mol. BioSyst., 2010, 6: 1503-1509). In addition, it has been reported that *c-myc* expression is elevated or deregulated in various human cancers and is associated with tumors (Oncogene, 1999, 18(19), 3004-16). Therefore, there has been much effort to develop anti-cancer agents or anti-tumor agents by regulating *c-myc* expression.

However, in development of related drugs, development of substances that directly inhibit c-Myc function has not been technically feasible, and thus most attempts have been made to indirectly regulate c-Myc function. However, such indirect c-Myc inhibitors may cause many unexpected side effects. In particular, since c-Myc plays an important role in regulating cellular activity in the body, serious side effects may occur when c-Myc inhibitors are not highly selective for c-Myc. In fact, development of many substances was discontinued due to toxicity problems. For example, JQ1 has recently been reported to be useful for myeloma treatment by indirectly regulating c-Myc expression (see Cell. 2011, 146(6): 904-917 and Blood. 2012, 120(14): 2843-2852), but development of JQ1 was discontinued due to serious side effects thereof.

Specifically, a motif responsible for binding of Myc and Max is the leucine zipper motif commonly found in general protein structures. Thus, certain proteins that bind to the leucine zipper motif inhibit Myc/Max heterodimer formation, but have low selectivity. In other words, when searching for a candidate substance, a substance that binds to a unique motif present in a Myc/Max heterodimer should be selected and selectivity thereof should be confirmed, or side effects may be caused. For example, certain c-Myc inhibitors exhibit low selectivity, inhibiting the activity of c-Jun/Fos transcription factors with similar structures. Therefore, it is important to develop an inhibitor capable of selectively acting on a Myc/Max heterodimer. In addition, a targeting substance that inhibits formation of a complex between a c-Myc/Max dimer and DNA may have higher selectivity than a targeting substance that inhibits c-Myc/Max dimer formation.

Accordingly, it is necessary to develop an inhibitor capable of directly inhibiting c-Myc action. Specifically, there is demand for the development of an inhibitor that has high selectivity for c-Myc and is capable of inhibiting c-Myc activity, thus reducing side effects.

WO 2008/102932 discloses anti-cancer compositions comprising certain 3-acetyl-2-aminoqunioline-4(1H)-one compounds Jeong Kyung-Chae et al, J. Urology, vol. 191, no. 2, pages 510-518 discloses that a compound KSI-3716 (the compound of Formula 4 below) blocks c-MYC/MAX from forming a complex with target gene promotors.

### [Disclosure]

### [Technical Problems]

Therefore, the present disclosure has been made in view of the above problems, and it is an objective of the present disclosure to provide novel compounds having inhibitory activity on c-Myc/Max/DNA complex formation.

### [Technical Solution]

The present disclosure provides compounds corresponding to Formula 3a or 3b below or pharmaceutically acceptable salts thereof: 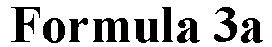 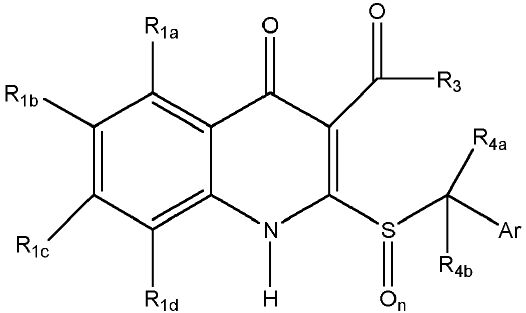 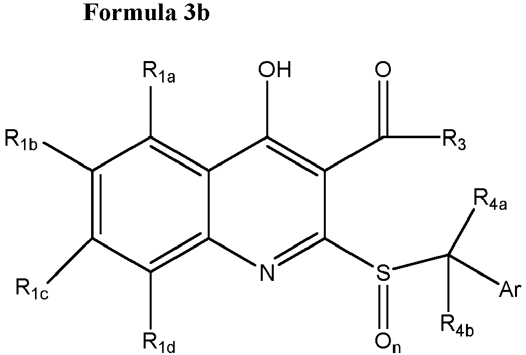 wherein,
R₁ₐ to R_{1d} are each independently hydrogen, a halogen, C₁₋₆ alkyl, C₁₋₆ haloalkyl, C₁₋₆ hydroxyalkyl, C₁₋₆ alkoxy, C₁₋₆ haloalkoxy, C₂₋₁₀ alkenyl, C₂₋₁₀ haloalkenyl, C₂₋₁₀ alkynyl, C₂₋₁₀ haloalkynyl, a hydroxyl group, nitro, cyano, C₁₋₆ alkoxycarbonyl, amino, C₁₋₆ alkylamino, di(C₁₋₆ alkyl)amino, amino(C₁₋₆)alkyl, (C₁₋₆)alkylamino(C₁₋₆)alkyl, C₁₋₆ alkanoyl, C₃₋₇ cycloalkyl, an aryl, a heterocycle, or a heteroaryl, wherein R₁ₐ to R_{1d} are each independently unsubstituted or optionally substituted;
R₃ is C₁₋₄ alkyl, isoalkyl, cycloalkyl, phenyl, or C₁₋₄ haloalkyl;
n is 1 or 2;
R₄ₐ and R_{4b} are each independently hydrogen, a halogen, C₁₋₄ alkyl, C₁₋₄ haloalkyl, or C₁₋₄ alkyl in which one or more hydrogens are substituted with substituents other than halogen ;
Ar is phenyl, heteroaryl being 5-6-membered and having heteroatoms selected independently from N, S, or O, or biheteroaryl being 8-12-membered and having heteroatoms selected independently from N, S, or O,
wherein Ar may be unsubstituted or may be optionally substituted with one or more of a halogen, C₁₋₆ alkyl, C₁₋₆ alkylthio, C₁₋₆ haloalkyl, C₁₋₆ haloalkylthio, C₁₋₆ alkoxy, C₁₋₆ haloalkoxy, C₂₋₁₀ alkenyl, C₂₋₁₀ haloalkenyl, C₂₋₁₀ alkynyl, C₂₋₁₀ haloalkynyl, a hydroxyl group, COOH, nitro, cyano, C₁₋₆ alkoxycarbonyl, amino, C₁₋₆ alkylamino, di(C₁₋₆ alkyl)amino, amino(C₁₋₆)alkyl, (C₁₋₆)alkylamino(C₁₋₆)alkyl, (C₁₋₆)alkoxy(C₁₋₆)alkylamino, (C₁₋₆)alkylamino(C₁₋₆) alkylamino, C₁₋₆ alkanoyl, SF₅, S(O)CF₃, SCF₃, NHC(=O)CH₃, C(=O)NHCH₃, NHSO2CH3, C₃₋₇ cycloalkyl, an aryl, benzoyl, a heterocycle, a heteroaryl, phenyl, oxazole, pyrazole, pyrrole, imidazole, thiazole, thiophene, pyridine, pyrimidine, furan, indole, benzopyrazole, benzothiazole, benzooxazole, isoxazole, benzoimidazole, or benzothiophene,
wherein the substituents of Ar may be unsubstituted or may be optionally substituted with one or more of CF₃, a halogen, (C₁₋₃)alkyl, (C₁₋₃)haloalkyl, hydrogen, COOH, nitro, cyano, amino, di(C₁₋₃ alkyl)amino, NHC(=O)CH₃, or C(=O)NHCH₃.

The invention also provides a compound as defined in claim 3.

In accordance with an aspect of the present disclosure, the above and other objectives can be accomplished by the provision of a (pharmaceutical) composition including compounds according to Formula 3a or 3b or pharmaceutically acceptable salts thereof, and a pharmaceutically acceptable carrier or additive.

In accordance with various aspects, the (pharmaceutical) composition may further include one or more additional pharmaceutically active agents.

Novel compounds of the present disclosure having structures of Formula 3a or 3b or pharmaceutically acceptable salts thereof are useful for inhibiting c-Myc/Max/DNA complex formation, and thus may be useful for treatment or prevention of cancers. Cancers, neoplasia, or tumors that can be treated by inhibiting c-Myc/Max/DNA complex formation include, for example, lung cancer (including small cell lung cancer and non-small cell lung cancer), colorectal cancer, colon cancer, rectal cancer, breast cancer, prostate cancer, bladder cancer, myeloma, leukemia, myelogenous leukemia, lymphoma, cervical carcinoma, osteosarcoma, glioblastoma, melanoma, pancreatic cancer, gastric cancer, liver cancer, kidney cancer, gallbladder cancer, biliary tract cancer, and esophageal cancer.

Novel compounds according to the present disclosure and a (pharmaceutical) composition including the compounds are described in detail as follows.

The following description is merely illustrative and is not intended to limit the present disclosure to specific application or uses.

As used herein, the following terms are defined as follows.

In the present specification, the terms "substituent", "radical", "group", "moiety", and "fragment" may be used interchangeably.

As used herein, the term "patient" refers to an animal (e.g., cow, horse, sheep, pig, chicken, turkey, quail, cat, dog, mouse, rat, rabbit or guinea pig), preferably a mammal such as a primate (e.g., monkey or human), and most preferably a human.

As used herein, the term "alkyl", when the number of carbon atoms is not particularly limited, refers to a saturated straight or branched non-cyclic hydrocarbon having 1 to 10 carbon atoms. The term "lower alkyl" refers to a straight or branched alkyl having 1 to 4 carbon atoms. Representative saturated straight alkyls include - methyl, -ethyl, -n-propyl, -n-butyl, -n-pentyl, -n-hexyl, -n-heptyl, -n-octyl, -n-nonyl and -n-decyl, whereas saturated branched alkyls include-isopropyl, -sec-butyl, -isobutyl, -tert-butyl, isopentyl, 2-methylhexyl, 3-methylbutyl, 2-methylpentyl, 3-methylpentyl, 4-methylpentyl, 2-methylhexyl, 3-methylhexyl, 2-methylpentyl, 3-methylpentyl, 4-methylpentyl, 2-methylhexyl, 3-methylhexyl, 4-methylhexyl, 5-methylhexyl, 2,3-dimethylbutyl, 2,3-dimethylpentyl, 2,4-dimethylpentyl, 2,3-dimethylhexyl, 2,4-dimethylhexyl, 2,5-dimethylhexyl, 2,2-dimethylpentyl, 2,2-dimethylhexyl, 3,3-dimethylpentyl, 3,3-dimethylhexyl, 4,4-dimethylhexyl, 2-ethylpentyl, 3-ethylpentyl, 2-ethylhexyl, 3-ethylhexyl, 4-ethylhexyl, 2-methyl-2-ethylpentyl, 2-methyl-3 -ethylpentyl, 2-methyl-4-ethylpentyl, 2-methyl-2-ethylhexyl, 2-methyl-3-ethylhexyl, 2-methyl-4-ethylhexyl, 2,2-diethylpentyl, 3,3-diethylhexyl, 2,2-diethylhexyl, and 3,3-diethylhexyl.

In the present specification, "C₁₋₆" means 1 to 6 carbon atoms. For example, C₁₋₆ alkyl means an alkyl having 1 to 6 carbon atoms.

As used herein, the term "alkenyl" refers to a saturated straight or branched non-cyclic hydrocarbon having 2 to 10 carbon atoms and including at least one carbon-carbon double bond. Representative straight and branched (C₂-C₁₀) alkenyls include -vinyl, -allyl, -1-butenyl, -2-butenyl, -isobutylenyl, -1-pentenyl, -2-pentenyl, -3-methy1-1-butenyl, -2-methyl-2-butenyl, -2,3-dimethyl-2-butenyl, -1-hexenyl, -2-hexenyl, -3-hexenyl, -1-heptenyl, -2-heptenyl, -3-heptenyl, -1-octenyl, -2-octenyl, -3-octenyl, -1-nonenyl, -2-nonenyl, -3-nonenyl, -1-decenyl, -2-decenyl, and -3-decenyl. These alkenyl groups may be optionally substituted. The term "cyclic alkylidene" is a ring having 3 to 8 carbon atoms and including at least one carbon-carbon double bond, and the ring may have 1 to 3 heteroatoms.

As used herein, the term "alkynyl" refers to a straight or branched non-cyclic hydrocarbon having 2 to 10 carbon atoms and including at least one carbon-carbon triple bond. Representative straight or branched (C₂-C₁₀) alkynyls include -acetylenyl, -propynyl, -1-butynyl, -2-butynyl, -1-pentynyl, -2-pentynyl, -3-methyl-1-butynyl, -4-pentynyl, -1-hexynyl, -2-hexynyl, -5-hexynyl, -1-heptynyl, -2-heptynyl, -6-heptynyl, -1-octynyl, -2-octynyl, -7-octynyl, -1-nonylyl, -2-nonylyl, -8-nonylyl, -1-decynyl, -2-decynyl, and -9-decynyl. These alkynyl groups may be optionally substituted.

As used herein, the terms "halogen" and "halo" refer to fluorine, chlorine, bromine or iodine.

As used herein, the term "haloalkyl", "haloalkoxy", "haloalkenyl" or "haloalkynyl" refers to an alkyl, alkoxy, alkenyl or alkynyl group wherein one or more hydrogen atoms are substituted with halogen atoms, respectively. For example, haloalkyls include -CF₃, -CHF₂, -CH₂F, -CBr₃, -CHBr₂, -CH₂Br, -CCl₃, -CHCl₂, -CH₂CI, -CI₃, -CHI₂, -CH₂I, -CH₂-CF₃, -CH₂-CHF₂, -CH₂-CH₂F, -CH₂-CBr₃, -CH₂-CHBr₂, -CH₂-CH₂Br, -CH₂-CCl₃, -CH₂-CHCl₂, -CH₂-CH₂CI, -CH₂-CI₃, -CH₂-CHI₂, -CH₂-CH₂I, and the like. Here, the alkyl and the halogen are as defined above.

As used herein, the term "alkanoyl" or "acyl" refers to -C(O)alkyl groups including -C(O)CH₃, -C(O)CH₂CH₃, -C(O)(CH₂)₂CH₃, -C(O)(CH₂)₃CH₃, -C(O)(CH₂)₄CH₃, -C(O)(CH₂)₅CH₃, and the like, wherein the alkyl is as defined above.

As used herein, the term "alkanoyloxy" or "acyloxy" refers to -OC(O)alkyl groups including -OC(O)CH₃, -OC(O)CH₂CH₃, -OC(O)(CH₂)₂CH₃, -OC(O)(CH₂)₃CH₃, -OC(O)(CH₂)₄CH₃, -OC(O)(CH₂)₅CH₃, and the like, wherein the alkyl is as defined above.

As used herein, the term "alkoxy" refers to -O-(alkyl) including -OCH₃, -OCH₂CH₃, -O(CH₂)₂CH₃, -O(CH₂)₃CH₃, -O(CH₂)₄CH₃, -O(CH₂)₅CH₃, and the like, wherein the alkyl is as defined above.

As used herein, the term "lower alkoxy" refers to -O-(lower alkyl), wherein the lower alkyl is as defined above.

As used herein, the term "aryl" refers to a carbocyclic aromatic group containing 5 to 10 cyclic atoms. Representative examples include phenyl, tolyl, xylyl, naphthyl, tetrahydronaphthyl, anthracenyl, fluorenyl, indenyl, azulenyl, and the like, without being limited thereto. The carbocyclic aromatic group may be optionally substituted.

The term "aryloxy" is RO-, wherein R is aryl as defined above. The term "arylthio" is RS-, wherein R is the aryl as defined above.

As used herein, the term "cycloalkyl" refers to a monocyclic or polycyclic saturated ring having carbon and hydrogen atoms and no carbon-carbon multiple bonds. For example, the cycloalkyl group includes (C₃-C₇)cycloalkyl (e.g., cyclopropyl, cyclobutyl, cyclopentyl, cyclohexyl and cycloheptyl), without being limited thereto. The cycloalkyl group may be optionally substituted. In one embodiment, the cycloalkyl group is a monocyclic or bicyclic ring.

As used herein, the term "mono-alkylamino" refers to -NH(alkyl) including - NHCH₃, -NHCH₂CH₃, -NH(CH₂)₂CH₃, -NH(CH₂)₃CH₃, -NH(CH₂)₄CH₃, -NH(CH₂)₅CH₃, and the like, wherein the alkyl is as defined above.

As used herein, the term "di-alkylamino" refers to N(alkyl)(alkyl) including - N(CH₃)₂, -N(CH₂CH₃)₂, -N((CH₂)₂CH₃)₂, -N(CH₃)(CH₂CH₃), and the like, wherein each alkyl is alkyl as defined above.

As used herein, the term "alkylamino" is a concept that includes mono-alkylamino and di-alkylamino as defined above.

As used herein, the terms "carboxyl" and "carboxy" refer to -COOH.

As used herein, the term "aminoalkyl" refers to -(alkyl)-NH₂ including -CH₂-NH₂, -(CH₂)₂-NH₂, -(CH₂)₃-NH₂, -(CH₂)₄-NH₂, -(CH₂)₅-NH₂, and the like, wherein the alkyl is as defined above.

As used herein, the term "mono-alkylaminoalkyl" refers to -(alkyl)-NH(alkyl) including -CH₂-NH-CH₃, -CH₂-NHCH₂CH₃, -CH₂-NH(CH₂)₂CH₃, -CH₂-NH(CH₂)₃CH₃, -CH₂-NH(CH₂)₄CH₃, -CH₂-NH(CH₂)₅CH₃, -(CH₂)₂-NH-CH₃, and the like, wherein each alkyl is alkyl as defined above.

As used herein, "heteroaryl" is a 5- to 10- membered aromatic heterocyclic ring that has at least one heteroatom selected from the group consisting of nitrogen, oxygen, and sulfur and that includes a mono- or bicyclic ring system and at least one carbon atom. Representative heteroaryls include triazolyl, tetrazolyl, oxadiazolyl, pyridyl, furyl, benzofuranyl, thiophenyl, benzothiophenyl, quinolinyl, pyrrolyl, indolyl, oxazolyl, benzoxazolyl, imidazolyl, benzimidazolyl, thiazolyl, benzothiazolyl, isoxazolyl, pyrazolyl, isothiazolyl, pyridazinyl, pyrimidinyl, pyrazinyl, triazinyl, cinnolinyl, phthalazinyl, quinazolinyl, pyrimidyl, oxetanyl, azepinyl, piperazinyl, morpholinyl, dioxanyl, tietanyl and oxazolyl.

As used herein, "heterocycle (heteroring)" refers to a saturated or unsaturated 5-to 7- membered monocyclic ring or a 7- to 10- membered bicyclic/heterocyclic ring containing 1 to 4 heteroatoms independently selected from nitrogen, oxygen and sulfur, wherein nitrogen and sulfur heteroatoms may be optionally oxidized, nitrogen heteroatoms may be optionally quaternized, and a bicyclic ring in which a part of the heterocycle is fused to a benzene ring is included. The heterocycle may be attached by heteroatoms or carbon atoms. The heterocycle includes the heteroaryl as defined above. Representative heterocycles include morpholinyl, pyrrolidinonyl, pyrrolidinyl, piperidinyl, hydantoinyl, valerolactamyl, oxiranyl, oxetanyl, tetrahydrofuranyl, tetrahydropyranyl, tetrahydropyridinyl, tetrahydropyrimidinyl, tetrahydrothiophenyl, tetrahydrothiopyranyl, tetrahydropyrimidinyl, tetrahydrothiophenyl, and tetrahydrothiopyranyl.

"Heterocycle fused to phenyl" refers to a heterocycle attached to two adjacent carbon atoms of a phenyl ring, wherein the heterocycle is as defined above.

As used herein, the term "hydroxyalkyl" refers to an alkyl in which one or more hydrogen atoms are substituted with hydroxy and includes -CH₂OH, -CH₂CH₂OH, -(CH₂)₂CH₂OH, -(CH₂)₃CH₂OH, -(CH₂)₄CH₂OH, -(CH₂)₅CH₂OH, -CH(OH)-CH₃, -CH₂CH(OH)CH₃, and the like, wherein the alkyl is as defined above.

As used herein, the term "sulfonyl" refers to -SO₃H.

As used herein, the term "sulfonylalkyl" refers to -SO₂-(alkyl) including -SO₂-CH₃, -SO₂-CH₂CH₃, -SO₂-(CH₂)₂CH₃, -SO₂-(CH₂)₃CH₃, -SO₂-(CH₂)₄CH₃, and -SO₂-(CH₂)₅CH₃, wherein the alkyl is as defined above.

As used herein, the term "sulfinylalkyl" refers to -SO-(alkyl) including -SO-CH₃, -SO-CH₂CH₃, -SO-(CH₂)₂CH₃, -SO-(CH₂)₃CH₃, -SO-(CH₂)₄CH₃, -SO-(CH₂)₅CH₃, and the like, wherein the alkyl is as defined above.

"Thioalkyl" includes -S-CH₃, -S-CH₂CH₃, -S-(CH₂)₂CH₃, -S-(CH₂)₃CH₃, -S-(CH₂)₄CH₃, -S-(CH₂)₅CH₃, and the like, wherein the alkyl is as defined above.

As used herein, the term "substituted" indicates that the hydrogen atom of the moiety (e.g., alkyl, aryl, heteroaryl, heterocycle or cycloalkyl) to be replaced is replaced with a substituent. In one embodiment, each carbon atom of the substituted group is not substituted with two or more substituents. In another embodiment, each carbon atom of the substituted group is not substituted with one or more substituents. In the case of a keto substituent, two hydrogen atoms are substituted with oxygen attached to carbon by a double bond.

Unless otherwise specified with respect to a substituent, a halogen, hydroxyl, (lower) alkyl, haloalkyl, mono- or di-alkylamino, aryl, heterocycle, -NO₂, -NRₐR_{b}, -NRₐC(=O)R_{b}, -NRₐC(=O)NRₐR_{b}, -NRₐC(=O)OR_{b}, -NRₐSO₂R_{b}, -ORₐ, -CN, -C(=O)Rₐ, -C(=O)ORₐ, -C(=O)NRₐR_{b}, -OC(=O)Rₐ, -OC(=O)ORₐ, -OC(=O)NRₐR_{b}, -NRₐSO₂R_{b}, -PO₃Rₐ, -PO(ORa)(ORb), -SO₂Rₐ, -S(O)Rₐ, -SO(NRₐ)R_{b} (e.g., sulfoximine), -S(NRₐ)R_{b} (e.g., sulfilimine) and -SRₐ may be used as substituents in the present disclosure, wherein Ra and R_{b} are the same or different and are each independently hydrogen, a halogen, amino, an alkyl, an alkoxyalkyl, a haloalkyl, aryl or a heterocycle, or may be in the form of a heterocycle containing attached nitrogen atoms. Here, Rₐ and R_{b} may be plural depending on the bonded atom.

As used herein, "basic structure of quinoline" refers to the following structure.

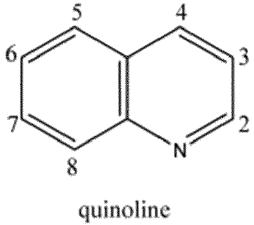

According to the present disclosure, "pharmaceutically acceptable salts" include salts of active compounds prepared from relatively non-toxic acids and bases depending on particular substituents found in the compounds described herein. When the compounds of the present disclosure include relatively acidic functionality, base addition salts may be obtained by bringing the neutral forms of the compounds into contact with a sufficient amount of a desired base in a pure or suitable inert solvent. For example, pharmaceutically acceptable base addition salts include sodium, potassium, calcium, ammonium, organic amino or magnesium salts or similar salts. When the compounds of the present disclosure include relatively basic functionality, acid addition salts may be obtained by bringing the neutral forms of the compounds into contact with a sufficient amount of a desired acid in a pure or suitable inert solvent. For example, pharmaceutically acceptable acid addition salts include salts derived from relatively non-toxic organic acids including acetic acid, propionic acid, isobutylic acid, oxalic acid, maleic acid, malonic acid, benzoic acid, succinic acid, suberic acid, fumaric acid, mandelic acid, phthalic acid, benzenesulfonic acid, p-tolylsulfonic acid, citric acid, tartaric acid, methanesulfonic acid, and analogs thereof. In addition, the pharmaceutically acceptable acid addition salts include hydrogen chloride, hydrogen bromide, nitric acid, carbonic acid, monohydrogen carbonic acid, phosphoric acid, monohydrogen phosphoric acid, dihydrogen phosphoric acid, sulfuric acid, monohydrogensulfuric acid, hydrogen iodide or phosphorous acid and analogs thereof. In addition, the pharmaceutically acceptable acid addition salts include salts of amino acids such as arginate and analogs thereof and analogs of organic acids such as glucuronic or galacturonic acids and analogs thereof (e.g., Berge et al. (1977) J. Pharm. Sci. 66: 1-19). Certain compounds of the present disclosure have both basic and acidic functionalities to convert the compounds into base or acid addition salts. Other examples of salts are disclosed in documents (e.g., Remington's Pharmaceutical Sciences, 18th eds., Mack Publishing, Easton PA (1990) or Remington : The Science and Practice of Pharmacy, 19th eds., Mack Publishing, Easton PA (1995)) known in the art to which the present disclosure pertains.

As used herein, "effective dose" refers to an amount of the compounds of the present disclosure sufficient to destroy, modify, control or eliminate primary, localized or metastatic cancer cells or cancer tissues; to slow or minimize the spread of cancer; or to provide therapeutic benefits in treatment or management of cancer, neoplastic diseases, or tumors. In addition, "effective dose" refers to an amount of the compounds of the present disclosure sufficient to cause the death of neoplastic cells including cancer cells. In addition, "effective dose" refers to an amount of the compounds sufficient to inhibit or reduce c-Myc/Max/DNA complex formation either *in vitro* or *in vivo.*

As used herein, "inhibition of c-Myc/Max/DNA complex formation" indicates that, when compared to cells that are not exposed to the compounds of the present disclosure, the amount of c-Myc/Max/DNA complexes is decreased or the degree of binding of the c-Myc/Max heterodimer to DNA is suppressed or delayed in cells exposed to the compounds of the present disclosure.

As used herein, "preventive effective dose" refers to an amount of the compounds of the present disclosure sufficient to inhibit cancer development in patients susceptible to the recurrence, or spread of cancer, susceptible to cancer or patients previously exposed to a carcinogen. At this time, the type of patient is not limited thereto.

As used herein, the term "neoplastic" refers to an abnormal growth of cells or tissues (e.g., a boil) that may be benign or cancerous.

As used herein, "prevention" refers to preventing the recurrence, spread or onset of cancer in a patient.

As used herein, "treatment" includes eradication, removal, modification, or control of primary, localized or metastatic cancer tissues; and refers to minimizing or delaying the spread of cancer.

As used herein, the term "the compounds of the present disclosure" refers to compounds of Formula 3 (3a and 3b), and also includes clathrates, hydrates, solvates, or polymorphs thereof. In addition, the term "the compounds of the present disclosure" also includes pharmaceutically acceptable salts of the compounds of the present disclosure, when pharmaceutically acceptable salts thereof are not mentioned. According to one embodiment, the compounds of the present disclosure may be present as stereomerically pure compounds (e.g., compounds that are substantially free of other stereoisomers (e.g., 85% ee or more, 90% ee or more, 95% ee or more, 97% ee or more, or 99% ee or more)). That is, in addition to the compounds corresponding to Formula 3, when the salts of the compounds are tautomeric isomers and/or stereoisomers (e.g., geometrical isomers and conformational isomers), isolated isomers thereof and respective mixtures thereof are within the scope of the compounds of the present disclosure. When the compounds of the present disclosure or salts thereof have asymmetric carbons in the structure thereof, optically active compounds and racemic mixtures thereof are also within the scope of the compounds of the present disclosure. For example, as shown in the following scheme, when the compounds of the present disclosure have a sulfoxide(SOR) structure, the compounds may have chirality. The R and S forms of these isomers are included in the category of the compounds of the present disclosure, and the mixtures of the R and S forms are also included in the category of the compounds of the present disclosure.

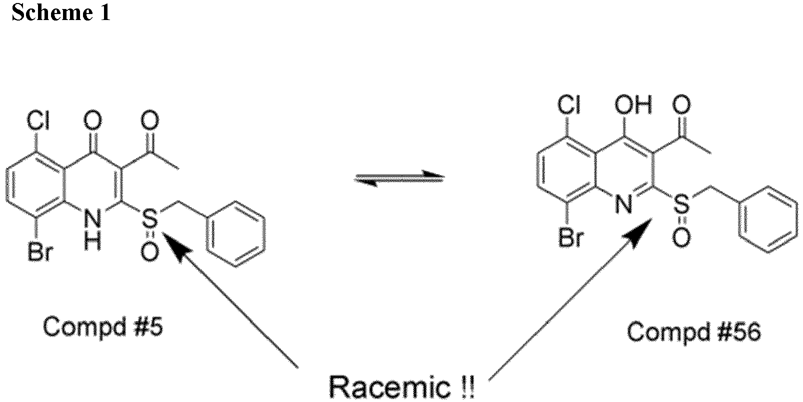

In addition, as shown in Scheme 1, the compounds of the present disclosure may exist in either keto or enol form, both of which are included in the category of the compounds of the present disclosure.

When used herein, the term "polymorph" refers to solid crystalline forms of the compounds of the present disclosure or complexes thereof. Different polymorphs of the same compound exhibit different physical, chemical and/or spectral characteristics. Differences in physical characteristics include stability (e.g., heat or light stability), compressibility and density (important for formulation and product production), and dissolution rate (which may affect bioavailability), without being limited thereto. Differences in stability cause changes in chemical reactivity (e.g., differential oxidation, as evidenced by more rapid color change when composed of one polymorph than when composed of another polymorph) or mechanical characteristics (e.g., as dynamically preferred polymorphs, stored tablet fragments are converted into more thermodynamically stable polymorphs), or both (tablets of one polymorph are more sensitive to degradation at high humidity). Other physical properties of polymorphs may affect processing thereof. For example, one polymorph may be more likely to form solvent compounds than another polymorph, e.g., due to a shape or particle size distribution thereof, or may be more difficult to filter or wash than another polymorph

As used herein, the term "solvent compounds" refers to the compounds of the present disclosure or pharmaceutically acceptable salts thereof, including a stoichiometric or non-stoichiometric amount of a solvent bound by force between non-covalent molecules. Preferred solvents are volatile and non-toxic, and may be administered to humans in very small doses.

As used herein, the term "hydrates" refers to the compounds of the present disclosure or pharmaceutically acceptable salts thereof, including a stoichiometric or non-stoichiometric amount of water bound by force between non-covalent molecules.

As used herein, the term "clathrates" refers to the compounds of the present disclosure or salts thereof in the form of a crystal lattice including a space (e.g., channel) in which guest molecules (e.g., solvent or water) are confined.

As used herein, the term "purified" indicates that when a substance is separated, the purity of the substance is at least 90%. The purity of the substance may be at least 95% in one embodiment, 99% in another embodiment, and 99.9% in still another embodiment.

The term "hydrido" refers to a single -H atom (H), and may be interchanged with the symbol "H" or the term "hydrogen".

When substituents are described as being "optionally substituted", the substituents may be unsubstituted (1) or may be substituted with at least one of substituents as defined (2). When a substitutable position is not substituted, a default substituent is a hydrido radical.

As used herein, the singular forms "a" and "an" may include the plural forms unless context clearly dictates otherwise.

The term "pharmaceutically acceptable" means suitable for use as a pharmaceutical preparation and is generally considered to be safe for such use. In addition, pharmaceutically acceptable substances refer to substances which have been formally approved by the governing body of the State for such use or which are on the list of Korean Pharmacopoeia or US Pharmacopoeia.

### Compounds of the present disclosure

The present disclosure provides compounds having the structures corresponding to Formula 3a or 3b or pharmaceutically acceptable salts thereof: 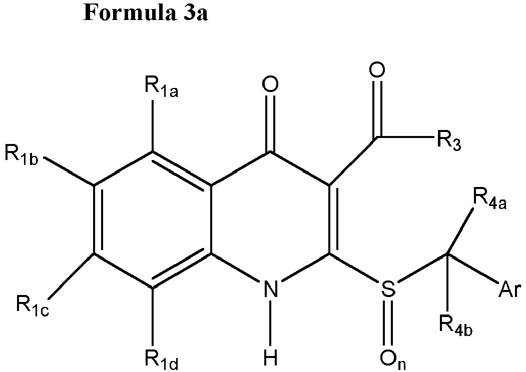 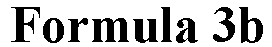 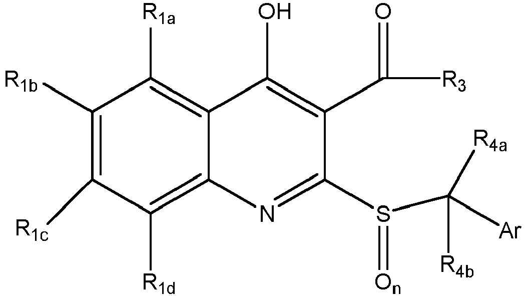 wherein
R₁ₐ to R_{1d} are each independently hydrogen, a halogen, C₁₋₆ alkyl, C₁₋₆ haloalkyl, C₁₋₆ hydroxyalkyl, C₁₋₆ alkoxy, C₁₋₆ haloalkoxy, C₂₋₁₀ alkenyl, C₂₋₁₀ haloalkenyl, C₂₋₁₀ alkynyl, C₂₋₁₀ haloalkynyl, a hydroxyl group, nitro, cyano, C₁₋₆ alkoxycarbonyl, amino, C₁₋₆ alkylamino, di(C₁₋₆ alkyl)amino, amino(C₁₋₆)alkyl, (C₁₋₆)alkylamino(C₁₋₆)alkyl, C₁₋₆ alkanoyl, C₃₋₇ cycloalkyl, an aryl, a heterocycle, or a heteroaryl, wherein R₁ₐ to R_{1d} are each independently unsubstituted or optionally substituted;
R₃ is C₁₋₄ alkyl, isoalkyl, cycloalkyl, phenyl, or C₁₋₄ haloalkyl;
n is 1 or 2;
R₄ₐ and R_{4b} are each independently hydrogen, a halogen, C₁₋₄ alkyl, C₁₋₄ haloalkyl, or C₁₋₄ alkyl in which one or more hydrogens are substituted with substituents other than halogen ;
Ar is phenyl, heteroaryl being 5-6-membered and having heteroatoms selected independently from N, S, or O, or biheteroaryl being 8-12-membered and having heteroatoms selected independently from N, S, or O,
wherein Ar may be unsubstituted or may be optionally substituted with one or more of a halogen, C₁₋₆ alkyl, C₁₋₆ alkylthio, C₁₋₆ haloalkyl, C₁₋₆ haloalkylthio, C₁₋₆ alkoxy, C₁₋₆ haloalkoxy, C₂₋₁₀ alkenyl, C₂₋₁₀ haloalkenyl, C₂₋₁₀ alkynyl, C₂₋₁₀ haloalkynyl, a hydroxyl group, COOH, nitro, cyano, C₁₋₆ alkoxycarbonyl, amino, C₁₋₆ alkylamino, di(C₁₋₆ alkyl)amino, amino(C₁₋₆)alkyl, (C₁₋₆)alkylamino(C₁₋₆)alkyl, (C₁₋₆)alkoxy(C₁₋₆)alkylamino, (C₁₋₆)alkylamino(C₁₋₆) alkylamino, C₁₋₆ alkanoyl, SF₅, S(O)CF₃, SCF₃, NHC(=O)CH₃, C(=O)NHCH₃, NHSO2CH3, C₃₋₇ cycloalkyl, an aryl, benzoyl, a heterocycle, a heteroaryl, phenyl, oxazole, pyrazole, pyrrole, imidazole, thiazole, thiophene, pyridine, pyrimidine, furan, indole, benzopyrazole, benzothiazole, benzooxazole, isoxazole, benzoimidazole, or benzothiophene,
wherein the substituents of Ar may be unsubstituted or may be optionally substituted with one or more of CF₃, a halogen, (C₁₋₃)alkyl, (C₁₋₃)haloalkyl, hydrogen, COOH, nitro, cyano, amino, di(C₁₋₃ alkyl)amino, NHC(=O)CH₃, or C(=O)NHCH₃.

To obtain novel compounds having high inhibitory activity on c-Myc/Max/DNA complex formation, having high selectivity to c-Myc/Max/DNA complexes, and consequently having an inhibitory effect on cancer cells while having minimal side effects, the present inventors synthesized various compounds and performed various experiments to evaluate the compounds. As a result, the present inventors completed the present disclosure by confirming that the novel compounds of the present disclosure were suitable for the above-described various objects.

For example, a compound having a substituent linked to -S- at the 2-position of the basic structure of quinoline is superior in safety to a compound having a substituent linked to -NH-. Specifically, in the case of the compound having a substituent linked to -NH-, the compound is somewhat superior but has very severe cardiotoxicity. For example, in the case of a mouse xenograft model experiment using compound KSI-3716 of the following Formula 4, all of the experimental group (30 mpk, intraperitoneal administration (IP)) died. On the other hand, when the compounds of the present disclosure (e.g., Compound 4) were subjected to IV and IP single toxicity tests using 40 mpk, there were no deaths, no significant weight changes, and no abnormal symptoms in terms of general symptoms such as feed intake and drinking water intake.

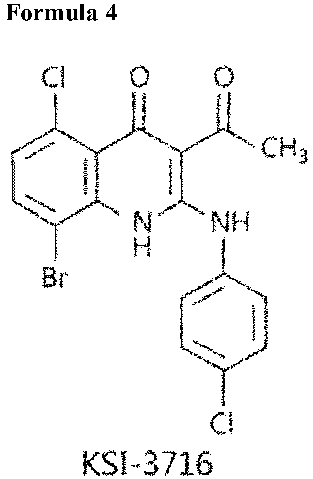

**[Table 1]**

| Single toxicity evaluation results of Compound 4 according to the present disclosure | | | |
|---|---|---|---|
| Species | Sex | Dose | Percentage |
| Mouse | Male | IV 40 mg/kg | 0 (0/2) |
| Mouse | Male | IP 40 mg/kg | 0 (0/2) |
| Mouse | Female | IV 40 mg/kg | 0 (0/2) |
| Mouse | Female | IP 40 mg/kg | 0 (0/2) |

| | | | |
|---|---|---|---|
| - No deaths were observed in single-dose administration of 40mpk. - General symptoms: Feeding and drinking were good, and no other abnormal symptoms were observed. - Weight changes: In general, weight gain was observed, but weight gain was slightly reduced in certain individuals. - In the case of KSI-3716, all animals died at 30mpk (IP). | | | |

In addition, in cardiotoxicity experiments using zebrafish, all zebrafish (n=10) died when 5 µM of a compound (e.g., KSI-3716 of Formula 4) having a substituent linked to -NH- was used. On the other hand, when the compounds of the present disclosure were used, lethality was very low and the compounds did not cause changes in heart rate. The experimental results of representative compounds are shown in the following Table 2.

**[Table 2]**

| Changes in heart rate depending on treatment of compounds in zebrafish (mean, n=10) | | | |
|---|---|---|---|
| Compounds | Changes in heart rate (%) | Lethality | Note |
| 10 µM Astemizole | 46.2 | 0/10 | |
| 5 µM of KSI-3716 | - | 10/10 | Not measurable by death of all zebrafish |
| 5 µM of Compound **4** | 88.5 | 1/10 | |
| 5 µM of Compound **33** | 97.3 | 0/10 | No significant heart rate inhibition |

For example, in view of activity, one or more of R₁ₐ to R_{1d} are preferably substituted with substituents, more preferably halogens. In particular, when R₁ₐ and R_{1d} were simultaneously substituted with halogens, even better activity was observed.

In the case of R₃, C₁₋₄ alkyl, isoalkyl, cycloalkyl, phenyl, or C₁₋₄ haloalkyl exhibited excellent activity. In particular, groups such as methyl or halomethyl exhibited better activity. In addition, when R₃ was -CF₃, metabolic stability was increased. On the other hand, when R₃ was heteroatoms of O or N (reference), the activity desired in the present disclosure was weak.

R₄ (R₄ₐ and/or R_{4b}) is an important site for metabolic stability, and is preferably a lower alkyl or halogen for various objects of the present disclosure.

Non-limiting examples of the compounds according to the present disclosure include the compounds of Table 3 below and pharmaceutically acceptable salts thereof.

**[Table 3]**

| Compoun d Number | Structure | IUPAC Name |
|---|---|---|
| 4 | 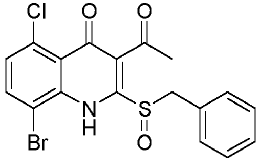 | 3-acetyl-2-(benzylsulfinyl)-8-bromo-5-chloroquinolin-4(1H)-one |
| 11 | 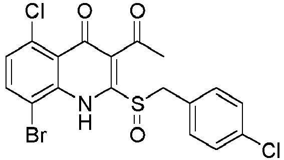 | 3-acetyl-8-bromo-5-chloro-2-((4-chlorobenzyl)sulfinyl)quinolin-4(1H)-one |
| 13 | 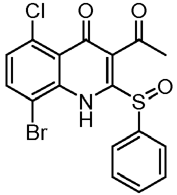 | 3-acetyl-8-bromo-5-chloro-2-(phenylsulfinyl)quinolin-4(1H)-one |
| 15 | 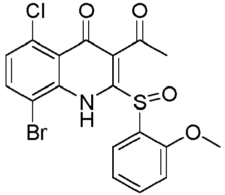 | 3-acetyl-8-bromo-5-chloro-2-((2-methoxyphenyl)sulfinyl)quinolin-4(1H)-one |
| 17 | 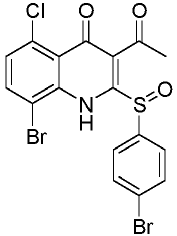 | 3-acetyl-8-bromo-2-((4-bromophenyl)sulfinyl)-5-chloroquinolin-4(1H)-one |
| 26 | 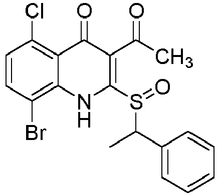 | 3-acetyl-8-bromo-5-chloro-2-((1-phenylethyl)sulfinyl)quinolin-4(1H)-one |
| 28 | 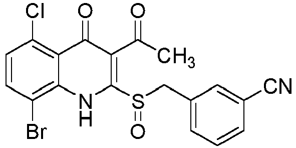 | 3-(((3-acetyl-8-bromo-5-chloro-4-oxo-1,4-dihydroquinolin-2-yl)sulfinyl)methyl)benzonitrile |
| 29 | 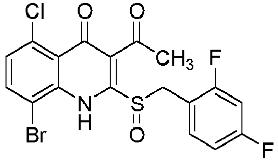 | 3-acetyl-8-bromo-5-chloro-2-((2,4-difluorobenzyl)sulfinyl)quinolin-4(1H)-one |
| 31 | 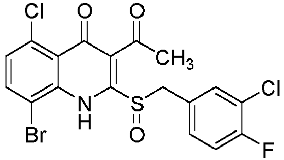 | 3-acetyl-8-bromo-5-chloro-2-((3-chloro-4-fluorobenzyl)sulfinyl)quinolin-4(1H)-one |
| 33 | 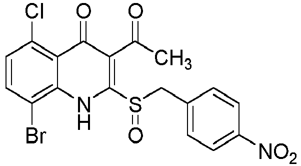 | 3-acetyl-8-bromo-5-chloro-2-((4-nitrobenzyl)sulfinyl)quinolin-4(1H)-one |
| 34 | 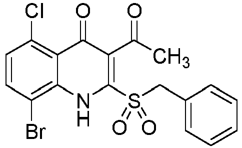 | 3-acetyl-2-(benzylsulfonyl)-8-bromo-5-chloroquinolin-4(1H)-one |
| 44 | 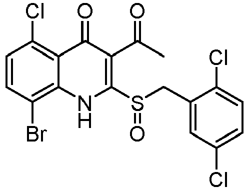 | 3-acetyl-8-bromo-5-chloro-2-((2,5-dichlorobenzyl)sulfinyl)quinolin-4(1H)-one |
| 46 | 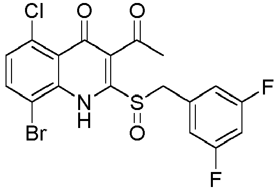 | 3-acetyl-8-bromo-5-chloro-2-((3,5-difluorobenzyl)sulfinyl)quinolin-4(1H)-one |
| 48 | 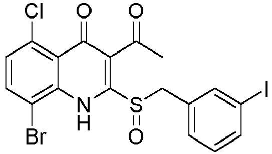 | 3-acetyl-8-bromo-5-chloro-2-((3-iodobenzyl)sulfinyl)quinolin-4(1H)-one |
| 50 | 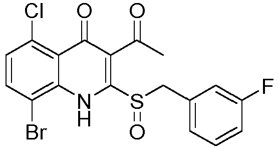 | 3-acetyl-8-bromo-5-chloro-2-((3-fluorobenzyl)sulfinyl)quinolin-4(1H)-one |
| 51 | 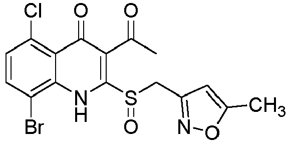 | 3-acetyl-8-bromo-5-chloro-2-(((5-methylisoxazol-3-yl)methyl)sulfinyl)quinolin-4(1H)-one |
| | | |
| | | |
| 53 | 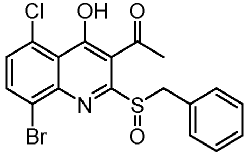 | 1-(2-(benzylsulfinyl)-8-bromo-5-chloro-4-hydroxyquinolin-3 -yl)ethan- 1 -one |
| 54 | 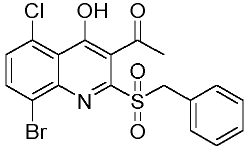 | 1-(2-(benzylsulfonyl)-8-bromo-5-chloro-4-hydroxyquinolin-3 -yl)ethan- 1 -one |
| 55 | 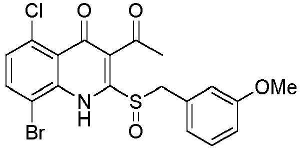 | 3-acetyl-8-bromo-5-chloro-2-((3-methoxybenzyl)sulfinyl)quinolin-4(1H)-one |
| 56 | 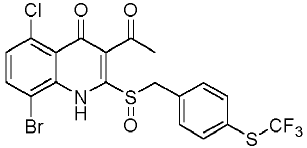 | 3-acetyl-8-bromo-5-chloro-2-((4-((trifluoromethyl)thio)benzyl)sulfinyl)quinolin -4(1H)-one |
| 57 | 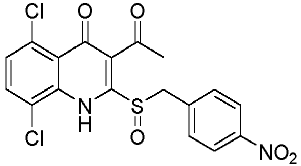 | 3-acetyl-5,8-dichloro-2-((4-nitrobenzyl)sulfinyl)quinolin-4(1H)-one |
| 58 | 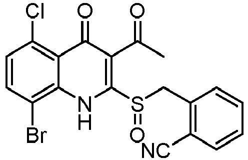 | 2-(((3-acetyl-8-bromo-5-chloro-4-oxo-1,4-dihydroquinolin-2-yl)sulfinyl)methyl)benzonitrile |
| 59 | 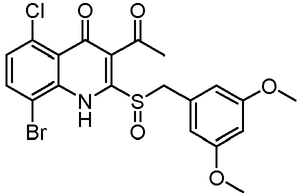 | 3-acetyl-8-bromo-5-chloro-2-((3,5-dimethoxybenzyl)sulfinyl)quinolin-4(1H)-one |
| 60 | 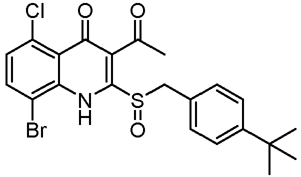 | 3-acetyl-8-bromo-2-((4-(tert-butyl)benzyl)sulfinyl)-5-chloroquinolin-4(1H)-one |
| 63 | 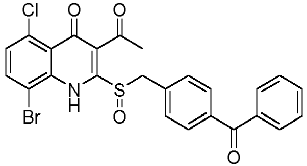 | 3-acetyl-2-((4-benzoylbenzyl)sulfinyl)-8-bromo-5-chloroquinolin-4(1H)-one |
| 64 | 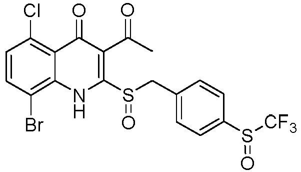 | 3-acetyl-8-bromo-5-chloro-2-((4-((trifluoromethyl)sulfinyl)benzyl)sulfinyl)quin olin-4(1H)-one |
| 68 | 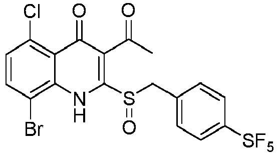 | 3-acetyl-8-bromo-5-chloro-2-((4-(pentafluoro-16-sulfanyl)benzyl)sulfinyl)quinolin-4(1H)-one |
| 69 | 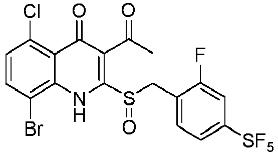 | 3-acetyl-8-bromo-5-chloro-2-((2-fluoro-4-(pentafluoro-16-sulfanyl)benzyl)sulfinyl)quinolin-4(1H)-one |
| 70 | 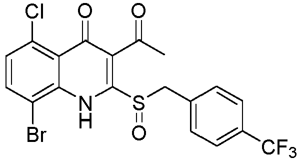 | 3-acetyl-8-bromo-5-chloro-2-((4-(trifluoromethyl)benzyl)sulfinyl)quinolin-4(1H)-one |
| 71 | 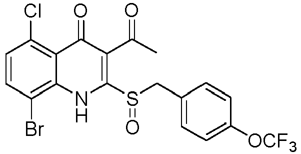 | 3-acetyl-8-bromo-5-chloro-2-((4-(trifluoromethoxy)benzyl)sulfinyl)quinolin-4(1H)-one |
| 72 | 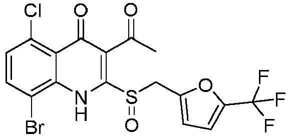 | 3-acetyl-8-bromo-5-chloro-2-(((5-(trifluoromethyl)furan-2-yl)methyl)sulfinyl)quinolin-4(1H)-one |
| 73 | 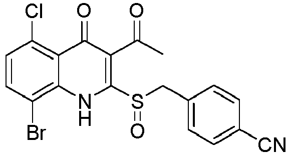 | 4-(((3-acetyl-8-bromo-5-chloro-4-oxo-1,4-dihydroquinolin-2-yl)sulfinyl)methyl)benzonitrile |
| 74 | 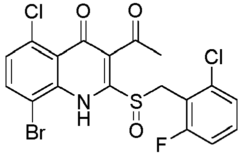 | 3-acetyl-8-bromo-5-chloro-2-((2-chloro-6-fluorobenzyl)sulfinyl)quinolin-4(1H)-one |
| 75 | 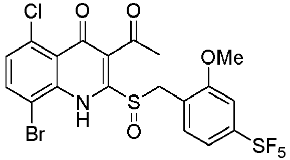 | 3-acetyl-8-bromo-5-chloro-2-((2-methoxy-4-(pentafluoro-λ6-sulfanyl)benzyl)sulfinyl)quinolin-4(1H)-one |
| 76 | 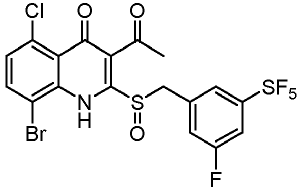 | 3 -acetyl-8-bromo-5 -chloro-2-((3 -fluoro-5 - (pentafluoro-λ6-sulfanyl)benzyl)sulfinyl)quinolin-4(1H)-one |
| 77 | 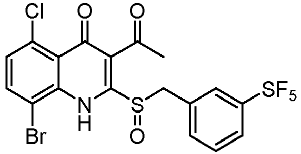 | 3-acetyl-8-bromo-5-chloro-2-((3-(pentafluoro-λ6-sulfanyl)benzyl)sulfinyl)quinolin-4(1H)-one |
| 78 | 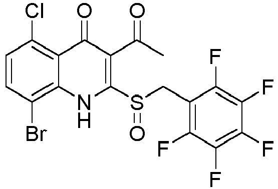 | 3-acetyl-8-bromo-5-chloro-2-(((perfluorophenyl)methyl)sulfinyl)quinolin-4(1H)-one |
| 79 | 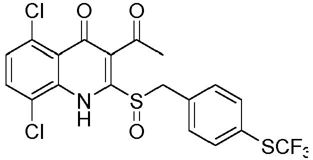 | 3-acetyl-5,8-dichloro-2-((4-((trifluoromethyl)thio)benzyl)sulfinyl)quinolin -4(1H)-one |
| 80 | 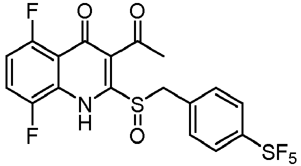 | 3-acetyl-5,8-difluoro-2-((4-(pentafluoro-λ6-sulfanyl)benzyl)sulfinyl)quinolin-4(1H)-one |
| 81 | 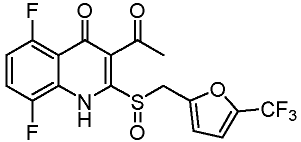 | 3-acetyl-5,8-difluoro-2-(((5-(trifluoromethyl)furan-2-yl)methyl)sulfinyl)quinolin-4(1H)-one |
| 82 | 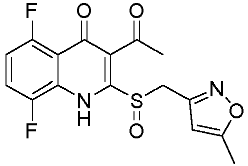 | 3-acetyl-5,8-difluoro-2-(((5-methylisoxazol-3 - yl)methyl)sulfinyl)quinolin-4(1H)-one |
| 83 | 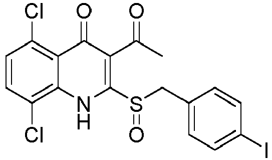 | 3-acetyl-5,8-dichloro-2-((4-iodobenzyl)sulfinyl)quinolin-4(1H)-one |
| 84 | 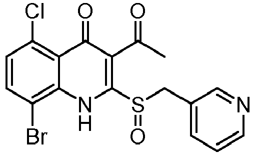 | 3-acetyl-8-bromo-5-chloro-2-((pyridin-3-ylmethyl)sulfinyl)quinolin-4(1H)-one |
| 85 | 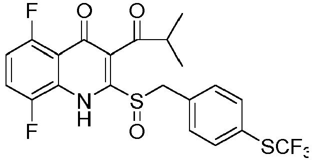 | 5,8-difluoro-3 -isobutyryl-2-((4-((trifluoromethyl)thio)benzyl)sulfinyl)quinolin -4(1H)-one |
| 86 | 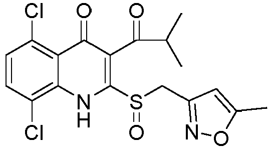 | 5,8 -dichloro-3 -isobutyryl-2-(((5 - methylisoxazol-3-yl)methyl)sulfinyl)quinolin-4(1H)-one |
| 87 | 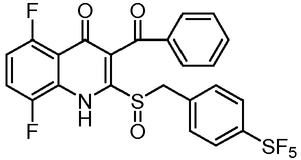 | 3-benzoyl-5,8-difluoro-2-((4-(pentafluoro-λ6-sulfanyl)benzyl)sulfinyl)quinolin-4(1H)-one |
| 88 | 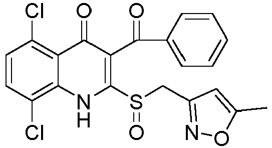 | 3-benzoyl- 5, 8-dichloro- 2-(((5-methylisoxazol-3-yl)methyl)sulfinyl)quinolin-4(1H)-one |
| 89 | 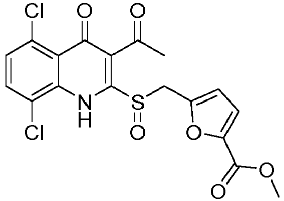 | methyl 5-(((3-acetyl-5,8-dichloro-4-oxo-1,4-dihydroquinolin-2-yl)sulfinyl)methyl)furan-2-carboxylate |
| 91 | 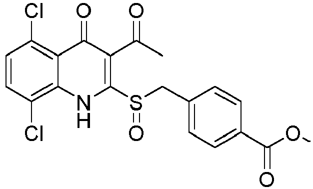 | methyl 4-(((3-acetyl-5,8-dichloro-4-oxo-1,4-dihydroquinolin-2-yl)sulfinyl)methyl)benzoate |
| 93 | 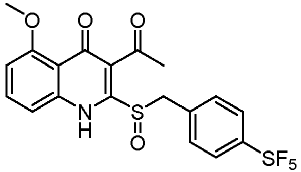 | 3-acetyl-5-methoxy-2-((4-(pentafluoro-λ6-sulfanyl)benzyl)sulfinyl)quinolin-4(1H)-one |
| 94 | 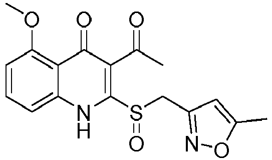 | 3 -acetyl-5 -methoxy-2-(((5 -methylisoxazol-3 - yl)methyl)sulfinyl)quinolin-4(1H)-one |
| 95 | 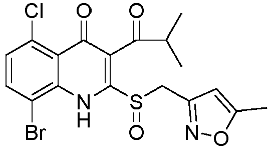 | 8-bromo-5-chloro-3-isobutyryl-2-(((5-methylisoxazol-3-yl)methyl)sulfinyl)quinolin-4(1H)-one |
| 96 | 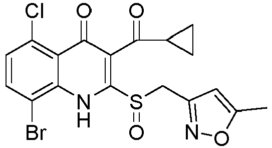 | 8-bromo-5-chloro-3-(cyclopropanecarbonyl)-2-(((5-methylisoxazol-3-yl)methyl)sulfinyl)quinolin-4(1H)-one |
| 97 | 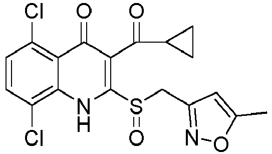 | 5,8-dichloro-3-(cyclopropanecarbonyl)-2-(((5 - methylisoxazol-3-yl)methyl)sulfinyl)quinolin-4(1H)-one |
| 98 | 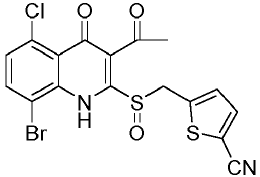 | 5-(((3-acetyl-8-bromo-5-chloro-4-oxo-1,4-dihydroquinolin-2-yl)sulfinyl)methyl)thiophene-2-carbonitrile |
| 99 | 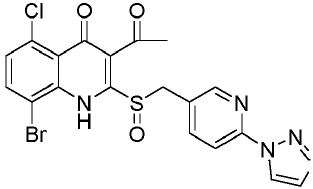 | 2-(((6-(1H-pyrazol-1-yl)pyridin-3-yl)methyl)sulfinyl)-3-acetyl-8-bromo-5-chloroquinolin-4(1H)-one |
| 100 | 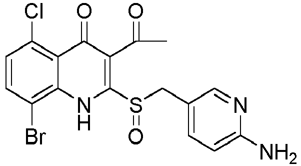 | 3-acetyl-2-(((6-aminopyridin-3-yl)methyl)sulfinyl)-8-bromo-5-chloroquinolin-4(1H)-one |
| 101 | 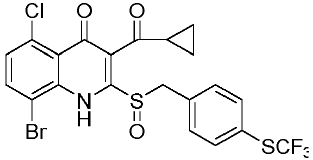 | 8-bromo-5-chloro-3-(cyclopropanecarbonyl)-2-((4-((trifluoromethyl)thio)benzyl)sulfinyl)quinolin -4(1H)-one |
| 102 | 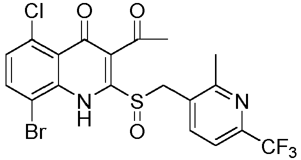 | 3-acetyl-8-bromo-5-chloro-2-(((2-methyl-6-(trifluoromethyl)pyridin-3-yl)methyl)sulfinyl)quinolin-4(1H)-one |
| 103 | 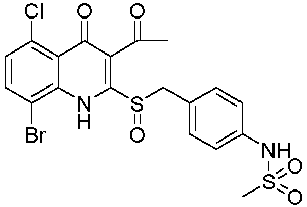 | N-(4-(((3 -acetyl-8 -bromo-5-chloro-4-oxo-1,4-dihydroquinolin-2-yl)sulfinyl)methyl)phenyl)methanesulfonamide |
| 104 | 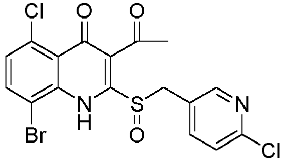 | 3-acetyl-8-bromo-5-chloro-2-(((6-chloropyridin-3-yl)methyl)sulfinyl)quinolin-4(1H)-one |
| 105 | 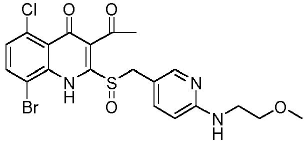 | 3-acetyl-8-bromo-5-chloro-2-(((6-((2-methoxyethyl)amino)pyridin-3-yl)methyl)sulfinyl)quinolin-4(1H)-one |
| 106 | 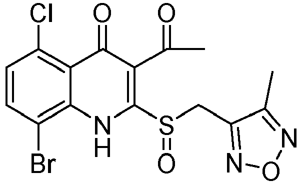 | 3-acetyl-8-bromo-5-chloro-2-(((4-methyl-1,2,5-oxadiazol-3-yl)methyl)sulfinyl)quinolin-4(1H)-one |
| 107 | 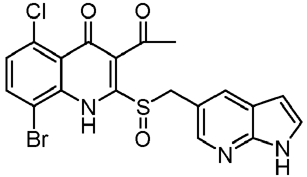 | 2-(((1H-pyrrolo[2,3-b]pyridin-5-yl)methyl)sulfinyl)-3-acetyl-8-bromo-5-chloroquinolin-4(1H)-one |

In another embodiment, the present disclosure provides therapeutically effective amounts of the compounds of Formula 3 or pharmaceutically acceptable salts thereof, and a (pharmaceutical) composition including a pharmaceutically acceptable carrier.

In another embodiment, the present disclosure provides therapeutically effective amounts of the compounds of Formula 3 or pharmaceutically acceptable salts thereof and a pharmaceutically acceptable carrier, and provides a (pharmaceutical) composition including a therapeutically effective amount of an active pharmaceutical ingredient selected from the group consisting of other anti-cancer agents other than the compounds of the present disclosure, cytostatic drugs, angiogenesis inhibitors, kinase inhibitors, cytokine blockers and cell adhesion molecule inhibitors.

When the novel compounds according to the present disclosure are used as anti-cancer agents, the dose is as follows. Any suitable route for administration of the compounds of the present disclosure may be selected, the type of pharmaceutical composition suitable for such route may be determined, and for the intended treatment, the compound may be administered in an effective dose. The effective dose is generally from about 0.001 to about 100 mg/kg body weight/day, preferably from about 0.01 to about 30 mg/kg/day, in a single or divided dose. Depending on the age, species, and diseases or conditions to be treated, a dose below the lower limit of this range may be appropriate. In other cases, higher doses may be used without harmful side effects. Higher doses may be divided into smaller doses and administered daily. Methods of determining an appropriate dose are well known in the art to which the present disclosure pertains. For example, a document, such as Remington: The Science and Practice of Pharmacy, Mack Publishing Co., 20th ed., 2000, may be used.

### References for preparing (pharmaceutical) composition

Methods for the preparation of pharmaceutical compositions for the treatment or prevention of diseases or conditions are well known to those of ordinary skill in the art. For example, as described in references such as Handbook of Pharmaceutical Excipients (7th ed.), Remington: The Science and Practice of Pharmacy (20th ed.), Encyclopedia of Pharmaceutical Technology (3rd ed.), Sustained and Controlled Release Drug Delivery Systems (1978), a pharmaceutically acceptable carrier, additives and the like may be suitably mixed with the compounds according to the present disclosure to prepare a pharmaceutical composition for the object of the present disclosure.

### [Advantageous Effects]

The present invention provides novel compounds capable of inhibiting c-Myc / Max / DNA complex formation and exhibiting various pharmacological activities. The compounds according to the present invention or their pharmaceutically acceptable salts are excellent in safety and have high selectivity in terms of inhibition of c-Myc / Max / DNA complex formation, so that they can exhibit various excellent effects.

### [Modes of the Invention]

Hereinafter, the present disclosure is described in detail with reference to the following examples. However, the examples according to the present disclosure can be modified into various other forms, and the scope of the present disclosure should not be construed as being limited to the following examples. The examples are provided to more fully explain the present disclosure to those skilled in the art to which the present disclosure pertains.

### Preparation of compounds of the present disclosure

The reagents and solvents used in the experiments described below can be purchased from Aldrich Chemical Co. (Milwaukee, Wisconsin, USA). A ¹H-NMR spectrum was measured using a Varian Gemini 400 MHz NMR spectrometer.

**Preparation of Compounds 3-acetyl-8-bromo-5-chloro-2-(methylthio)quinolin-4(1H)-one (4a), 3-acetyl-2-(benzylthio)-8-bromo-5-chloroquinolin-4(1H)-one (4b), 3-acetyl-8-bromo-5-chloro-2-(methylsulfinyl)quinolin-4(1H)-one (5a), and 3-acetyl-2-(benzylsulfinyl)-8-bromo-5-chloroquinolin-4(1H)-one (5b)**

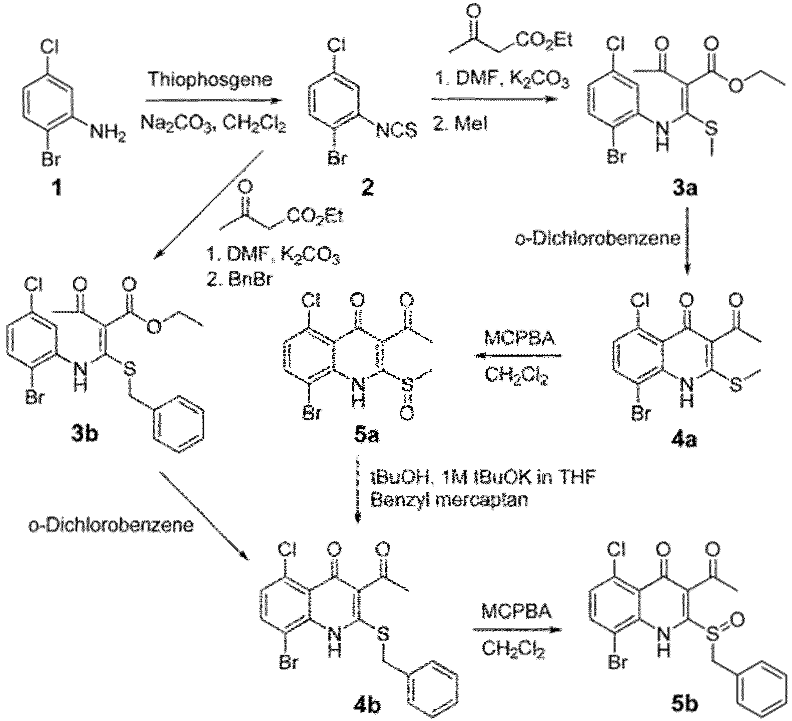

### Synthesis of 2-bromo-5-chlorophenyl isothiocyanate (2) (isothiocyanate formation)

2-Bromo-5-chloroaniline (**1**) (10 g, 48.5 mmol) was dissolved in anhydrous dichloroethane (CH₂Cl₂, 250 mL) and sodium carbonate (Na₂CO₃, 11 g, 97 mmol) was added thereto. The solution was cooled to 5°C with ice water under nitrogen gas, and thiophosgene (5.5 mL, 72.7 mmol) was added very slowly to the solution in that state. The reaction solution was stirred at room temperature for 12 hours and then filtered to remove inorganic matter. After removing the solvent by distillation under reduced pressure, nucleic acid (n-Hexane, 50 mL) was added to the resulting solid, and then the mixture was stirred for 10 minutes and subjected to filtering to quantitatively obtain a title compound as a yellow solid.

¹H NMR (300 MHz, CDCl₃) δ 7.51-7.49(d, *J*=8.61 Hz, 1H), 7.26-7.25(d, *J*=2.4 Hz, 1H), 7.13-7.09(dd, *J*=2.46, 6.18 Hz, 1H).
LC/MS data: 249.52 g/mol

### Synthesis of ethyl (Z)-2-(((2-bromo-5-chlorophenyl)amino)(methylthio)methylene)-3-oxobutanoate, Compound 3a (C=C bond formation)

Isothiothianate (2) (10 g, 40 mmol) synthesized in step 1 was dissolved in anhydrous DMF (20 mL), and the mixed solution was slowly added to a solution of ethyl oxobutanoate (5.2 g, 40 mmol) and K₂CO₃ (5.6 g, 40 mmol) dissolved in DMF (100 mL) at room temperature. The mixture was stirred for 12 hours at room temperature, and then iodomethane (5.7 g, 40 mmol) was slowly added thereto at room temperature. The solution was then stirred at room temperature for one day. After completion of the reaction was confirmed by TLC, water and ethyl acetate were added and the desired compound was extracted as an organic layer. Water was removed from the extracted organic layer using MgSO₄, and the extracted organic layer was subjected to distillation under reduced pressure, and then purification was performed using a column to obtain title Compound 3a.

¹H NMR (300 MHz, CDCl₃) δ 12.90(s, 1H), 7.45-7.42(d, *J*=8.41 Hz, 1H), 6.90-6.86(d, *J*=7.74 Hz, 1H), 6.68(s, 1H), 4.36-4.29(m, 2H), 2.54(s, 3H), 2.04(s, 3H), 1.37-1.33(t, *J*=7.26 Hz, 3H).
LC/MS data: 393.69 g/mol

### Synthesis of 3-acetyl-8-bromo-5-chloro-2-(methylthio)quinolin-4(1H)-one, Compound 4a (Cyclization)

Compound **3a** synthesized in step 2 was dissolved in o-dichlorobenzene and stirred for 12 hours while heated at 180°C. After the reaction was completed, the reaction mixture was cooled to room temperature and was subj ected to distillation under reduced pressure. A nucleic acid was added to the resulting solid, and the mixture was stirred for 10 minutes and was subjected to filtering to obtain Compound **4a.**

¹H NMR (300 MHz, CDCl₃) δ 8.67(s, 1H), 7.91-7.88(d, *J*=8.19 Hz, 1H), 7.71-7.68(d, *J*=8.49 Hz, 1H), 2.97(s, 3H), 2.79(s, 3H).
LC/MS data: 347.62 g/mol

### Synthesis of 3 -acetyl-8-bromo-5-chloro-2-(methylsulfinyl)quinolin-4(1H)-one, Compound 5a (Oxidation)

The quinolone compound **4a** obtained in step 3 was oxidized with MCPBA (1.5 eq.) in anhydrous dichloroethane (CH₂Cl₂, 10 mL) to obtain title Compound **5a.**

¹H NMR (300 MHz, CDCl₃) δ 11.13(s, 1H), 7.82-7.79(d, *J*=8.43 Hz, 1H), 7.38-7.36(d, *J*=8.46 Hz, 1H), 3.02(s, 3H), 2.78(s, 3H).
LC/MS data: 363.62 g/mol

### Synthesis of 3-acetyl-2-(benzylsulfinyl)-8-bromo-5-chloroquinolin-4(1H)-one, Compound 5b

Title Compound **5b** was synthesized in a similar manner to the synthesis of Compound **5a.**

Using the above-mentioned methods, the following compounds according to the present disclosure were synthesized by modifying reactants and/or starting materials appropriately. LC/MS and ¹H NMR measurement results are summarized in Table 4. In Table 4 below, MW refers to an average molecular weight, and MS is the value obtained by analyzing the actually prepared compounds.

**[Table 4]**

| Compo und Numbe r | Formula Name | MW (Molec ular Weight ) | LC/ MS data | ¹H NMR |
|---|---|---|---|---|
| 4 | 3-acetyl-2-(benzylsulfinyl)-8-bromo-5-chloroquinolin-4(1H)-one | 438.72 | 439 | ¹H NMR (300 MHz, CDCl₃)δ10.24 (br, 1H), 7.67 (d, *J*= 8.4 Hz, 1H), 7.29 (d, *J*= 8.4 Hz, 1H), 7.13 -7.22 (m, 3H), 7.08 -7.11 (m, 2H), 4.59 - 4.25 (m, 2H), 2.84 (s, 3H). |
| 11 | 3-acetyl-8-bromo-5-chloro-2-((4-chlorobenzyl)sulfinyl)quinolin-4(1H)-one | 473.16 | 472 | ¹H NMR (300 MHz, CDCl₃)δ10.21(s, 1H), 7.7 3-7.71(d,J=8.43 Hz, 1H), 7.33-7.3 1(d, *J*=8.37 Hz, 1H), 7.18-7.15(d, J=9 Hz, 2H), 7.05-7.03(d, *J*=8.25 Hz, 2H), 4.37(s, 2H), 2.84(s, 3H). |
| 13 | 3-acetyl-8-bromo-5-chloro-2-(phenylsulfinyl)quinolin-4(1H)-one | 424.69 | 424 | ¹H NMR (300 MHz, CDCl₃)δ7.84-7.75(m,2H),7.68-7.64(m,2H),7.52-7.50(d,J=8.43 Hz, 1H), 7.48-7.45(m, 1H), 7.38-7.35(d, *J*=8.43 Hz, 1H), 2.78(s, 3H). |
| 15 | 3-acetyl-8-bromo-5-chloro-2-((2-methoxyphenyl)sulfinyl)quinolin-4(1H)-one | 454.72 | 454 | ¹H NMR (300 MHz, CDCl₃)δ7.84(d,*J* = 8.4 Hz, 1H), 7.383 (d, *J* = 8.4 Hz, 1H),7.53 (m, 1H), 7.415 (m, 1H), 6.949 (s, 1H), 6.956 (s, 1H), 3.86 (s, 3H), 2.634 (s, 3H) |
| 17 | 3-acetyl-8-bromo-2-((4-bromophenyl)sulfinyl)-5-chloroquinolin-4(1H)-one | 503.59 | 502 | ¹H NMR (300 MHz, CDCl₃)δ11.37(s,1H),7.8 4-7.81(d,*J*=8.46 Hz, 1H), 7.73-7.70(d, *J*=8.73 Hz, 2H), 7.60-7.57(d, *J*=8.67 Hz, 2H), 7.39-7.38(d, *J*=8.43 Hz, 1H), 2.70(s, 3H). |
| 26 | 3-acetyl-8-bromo-5-chloro-2-((1-phenylethyl)sulfinyl)quinolin-4(1H)-one | 452.75 | 452 | ¹H NMR (300 MHz, CDCl₃)δ11.06(br,1H),7.8 2(d,*J*= 8.4 Hz, 1H), 7.60-7.65 (m, 2H), 7.41-7.50 (m, 3H), 7.38 (d, *J*= 8.5 Hz, 1H), 4.72 (q, *J*= 7.3 Hz, 1H), 2.84 (s, 3H), 1.42 (d, *J*= 7.1 Hz, 3H). |
| 28 | 3-(((3-acetyl-8-bromo-5-chloro-4-oxo-1,4-dihydroquinolin-2-yl)sulfinyl)methyl)benzonitrile | 463.73 | 463 | ¹H NMR (300 MHz, CDCl₃)δ10.27(br, 1H),7.7 2(d,*J*= 8.5 Hz, 1H), 7.53-7.57 (m, 1H), 7.27-7.40 (m, 2H), 4.59 - 4.22 (m, 2H), 2.85 (s, 3H). |
| 29 | 3-acetyl-8-bromo-5-chloro-2-((2,4-difluorobenzyl)sulfinyl)quinolin-4(1H)-one | 474.70 | 474 | ¹H NMR (300 MHz, CDCl₃)δ10.34(br,1H),7.7 4(d,*J*= 8.4 Hz, 1H), 7.35 (d, *J*= 8.4 Hz, 1H), 7.20-7.28 (m, 1H), 6.81-6.87 (m, 1H), 6.60-6.75 (m, 1H), 4.53 (dd, *J*= 48.9, 13.2 Hz, 2H), 2.85 (s, 3H). |
| 31 | 3-acetyl-8-bromo-5-chloro-2-((3-chloro-4-fluorobenzyl)sulfinyl)quinolin-4(1H)-one | 491.15 | 490 | ¹H NMR (300 MHz, CDCl₃)δ10.27(br, 1H),7.7 3(d,*J*= 8.4 Hz, 1H), 7.33 (d, *J*= 8.4 Hz, 1H), 7.22-7.24 (m, 1H), 6.95-6.98 (m, 2H), 4.33 (q, *J*= 12.8 Hz, 2H), 2.84 (s, 3H). |
| 33 | 3-acetyl-8-bromo-5-chloro-2-((4-nitrobenzyl)sulfinyl)quinolin-4(1H)-one | 483.72 | 483 | ¹H NMR (300 MHz, CDCl₃)δ10.23(br, 1H),8.0 8(d,*J*= 8.7 Hz, 2H), 7.70 (d, *J*= 8.4 Hz, 1H), 7.34 (dd, *J*= 8.5, 3.9 Hz, 3H), 4.49 (dd, *J*= 27.8, 12.6 Hz, 2H), 2.85 (s, 3H). |
| 34 | 3-acetyl-2-(benzylsulfonyl)-8-bromo-5-chloroquinolin-4(1H)-one | 454.72 | 454 | ¹H NMR (300 MHz, CDCl₃)δ10.25(s, 1H), 7.6 6(d,J=8.46 Hz, 1H), 7.28(d, *J*=8.43 Hz, 1H), 7.20-7.08(m, 5H), 4.41(d, *J*=3, 2H), 2.84(s, 3H). |
| 44 | 3-acetyl-8-bromo-5-chloro-2-((2,5-dichlorobenzyl)sulfinyl)quinolin-4(1H)-one | 507.60 | 506 | ¹H NMR (300 MHz, CDCl₃)δ10.35(s,1H),7.7 3-7.72(d,J=10.86 Hz, 1H), 7.32(s, 2H), 7.17(s, 2H), 4.81-4.49(dd, *J*= 13.33, 69.53 Hz, 2H), 2.81(s, 3H). |
| 46 | 3-acetyl-8-bromo-5-chloro-2-((3,5-difluorobenzyl)sulfinyl)quinolin-4(1H)-one | 474.70 | 474 | ¹H NMR (300 MHz, CDCl₃)δ10.43(s,1H),7.7 5-7.72(d,J= 8.4 Hz, 1H), 7.35-7.32(d. *J*= 8.46 Hz, 1H), 6.80-6.68(m, 3H), 4.44-4.23(q, *J*= 12.63, 36.84 Hz, 2H), 2.84(s, 3H). |
| 48 | 3-acetyl-8-bromo-5-chloro-2-((3-iodobenzyl)sulfinyl)quinolin-4(1H)-one | 564.62 | 564 | ¹H NMR (300 MHz, CDCl₃)δ10.19(s,1H),7.7 4-7.71(d,*J*=8.46 Hz, 1H), 7.56-7.54(d, *J*=8.07 Hz, 1H), 7.39(s, 1H), 7.35-7.32(d, *J*=8.46 Hz, 1H), 7.09-7.07(d, *J*=8.01 Hz, 1H), 6.97-6.92(t, *J*= 7.74 Hz, 1H), 4.40-4.30(q, *J*=12.71, 6.06 Hz, 2H), 2.87(s, 3H). |
| 50 | 3-acetyl-8-bromo-5-chloro-2-((3-fluorobenzyl)sulfinyl)quinolin-4(1H)-one | 456.71 | 456 | ¹H NMR (300 MHz, CDCl₃)δ10.30(s, 1H),7.7 0-7.67(d,J=8.56 Hz, 1H), 7.30-7.27(d, *J*=8.43 Hz, 1H), 7.15-7.07(m, 1H), 6.98-6.88(m, 2H), 6.83-6.81(d, *J*=7.68 Hz, 1H), 4.42-4.32(q, J=12.66, 3.33 Hz, 2H), 2.82(s, 3H). |
| 51 | 3-acetyl-8-bromo-5-chloro-2-(((5-methylisoxazol-3-yl)methyl)sulfinyl)quinolin-4(1H)-one | 443.70 | 442 | ¹H NMR (300 MHz, CDCl₃)δ10.50(br, 1H),7.7 7(d,*J*= 8.4 Hz, 1H), 7.36 (d, *J*= 8.4 Hz, 1H), 6.17 (s, 1H), 4.33-4.67 (m, 2H), 2.83 (s, 3H), 2.39 (s, 3H). |
| 53 | 1-(2-(benzylsulfinyl)-8-bromo-5-chloro-4-hydroxyquinolin-3-yl)ethan-1-one | 438.72 | 438 | ¹H NMR (300 MHz, CDCl₃)δ7.99(d,*J*= 8.2 Hz, 1H), 7.60 (s, 1H), 7.54-7.57 (m, 2H), 7.51 (d, *J*= 8.2 Hz, 1H), 7.37-7.48 (m, 3H), 5.39 (s, 2H), 3.48 (s, 3H). |
| 54 | 1-(2-(benzylsulfonyl)-8-bromo-5-chloro-4-hydroxyquinolin-3-yl)ethan-1-one | 454.72 | 454 | ¹H NMR (300 MHz, CDCl₃)δ7.94(d,*J*= 8.2 Hz, 1H), 7.73 (s, 1H), 7.54-7.58 (m, 2H), 7.45 (d, *J*= 8.1 Hz, 2H), 7.38-7.41 (m, 2H), 5.42 (s, 2H), 3.01 (s, 3H). |
| 55 | 3-acetyl-8-bromo-5-chloro-2-((3-methoxybenzyl)sulfinyl)quinolin-4(1H)-one | 468.75 | 468 | ¹H NMR (300 MHz, CDCl₃)δ10.32(s,1H),7.7 1-7.69(d,*J*=8.4 Hz, 1H), 7.33-7.30(d, J=8.43, 1H), 7.09-7.04(t, *J*=7.8 Hz,1H), 6.76-6.70(m, 2H), 6.65-6.63(d, *J*=7.47 Hz, 1H), 4.44-4.34(q, *J*=12.7, 4.86 Hz, 2H), 3.70(s, 3H), 2.86(s, 3H). |
| 56 | 3-acetyl-8-bromo-5-chloro-2-((4-((trifluoromethyl)thio)benzyl)sulfiny l)quinolin-4(1H)-one | 538.78 | 538 | ¹H NMR (300 MHz, CDCl₃)δ10.27(s,1H), 7.6 9-7.66(d,J=8.40 Hz, 1H), 7.49-7.46(d, *J*=8.01 Hz, 2H), 7.31-7.28(d, *J*=8.40 Hz, 1H), 7.18-7.15(d, *J*=8.16 Hz, 2H), 4.52-4.37(q, *J*=12.7, 17.1 Hz, 2H), 2.84(s, 3H). |
| 57 | 3-acetyl-5,8-dichloro-2-((4-nitrobenzyl)sulfinyl)quinolin-4(1H)-one | 439.26 | 439 | ¹H NMR (300 MHz, MeOD) δ9.95(s, 1H), 8.22-8.20(d, *J*=8.6 Hz, 2H), 7.73-7.70(d, J=8.31 Hz, 2H), 7.63-7.60(d, *J*=8.22 Hz,1H), 7.29-7.26(d, *J*=8.13 Hz, 1H), 4.59-4.10(q, J=12.3, 122 Hz, 2H), 2.72(s, 3H). |
| 58 | 2-(((3-acetyl-8-bromo-5-chloro-4-oxo-1,4-dihydroquinolin-2-yl)sulfinyl)methyl)benzonitrile | 463.73 | 463 | ¹H NMR (300 MHz, CDCl₃)δ10.30(br,1H),7.6 9(d,*J*= 8.4 Hz, 1H), 7.47 - 7.61 (m, 2H), 7.34 - 7.45 (m, 2H), 7.31 (d, *J*= 8.4 Hz, 1H), 4.71 (q, *J*= 13.1 Hz, 2H), 2.85 (s, 3H) |
| 59 | 3-acetyl-8-bromo-5-chloro-2-((3,5-dimethoxybenzyl)sulfinyl)quinolin-4(1H)-one | 498.77 | 498 | ¹H NMR (300 MHz, CDCl₃)δ10.36(s,1H),7.6 9(d,*J*= 8.4 Hz, 1H), 7.30 (d, *J*= 8.4 Hz, 1H), 6.23 (s, 3H), 4.25-4.39 (m, 2H), 3.62 (s, 6H), 2.83 (s, 3H). |
| 60 | 3-acetyl-8-bromo-2-((4-(tert-butyl)benzyl)sulfinyl)-5 - chloroquinolin-4(1H)-one | 494.83 | 494 | ¹H NMR (300 MHz, CDCl₃)δ10.15(br,1H),7.6 2(d,*J*= 8.4 Hz, 1H), 7.25 (d, *J*= 8.4 Hz, 1H), 7.13 (d, *J*= 8.3 Hz, 2H), 6.96 (d, *J*= 8.3 Hz, 2H), 4.40 (dd, *J*= 53.3, 12.7 Hz, 2H),, 2.83 (s, 3H), 1.11 (s, 9H). |
| 63 | 3-acetyl-2-((4-benzoylbenzyl)sulfinyl)-8-bromo-5-chloroquinolin-4(1H)-one | 542.83 | 542 | ¹H NMR (300 MHz, CDCl₃)δ10.29(br,1H),7.6 7(d,*J*= 8.4 Hz, 1H), 7.61 (d, *J*= 8.2 Hz, 2H), 7.37 - 7.59 (m, 5H), 7.31 (d, *J*= 8.4 Hz, 1H), 7.23 (d, *J*= 8.2 Hz, 2H), 4.51 (q, *J*= 12.6 Hz, 2H), 2.85 (s, 3H). |
| 64 | 3-acetyl-8-bromo-5-chloro-2-((4-((trifluoromethyl)sulfinyl)benzyl)sul finyl)quinolin-4(1H)-one | 554.78 | 554 | ¹H NMR (300 MHz, CDCl₃)δ10.30 (br s, 1H), 7.70-7.62(m, 3H), 7.41-7.26(m, 3H), 4.55-4.45(q, *J*= 16.3 Hz, 2H), 2.84 (s, 3H). |
| 68 | 3-acetyl-8-bromo-5-chloro-2-((4-(pentafluoro-16-sulfanyl)benzyl)sulfinyl)quinolin-4(1H)-one | 564.76 | 564 | ¹H NMR (300 MHz, CDCl₃)δ10.14 (s, 1H), 7.71 (d, *J*= 8.4 Hz, 1H), 7.57 (d, *J*= 8.7 Hz, 2H), 7.33 (d, *J*= 8.5 Hz, 1H), 7.20 (d, *J*= 8.3 Hz, 2H), 4.47 (dd, *J*= 30.2, 12.6 Hz, 2H), 2.87 (s, 3H). |
| 69 | 3-acetyl-8-bromo-5-chloro-2-((2-fluoro-4-(pentafluoro-16-sulfanyl)benzyl)sulfinyl)quinolin-4(1H)-one | 582.75 | 582 | ¹H NMR (300 MHz, CDCl₃)δ10.19(s,1H),7.7 1-7.68(d,*J*=8.43 Hz, 1H), 7.33-7.30(d, *J*=8.31 Hz, 1H), 4.73-4.40(q, *J*=13.0, 72.1 Hz, 2H), 2.83(s, 3H). |
| 70 | 3-acetyl-8-bromo-5-chloro-2-((4-(trifluoromethyl)benzyl)sulfinyl)qui nolin-4(1H)-one | 506.72 | 506 | ¹H NMR (300 MHz, CDCl₃)δ10.16 (s, 1H), 7.71 (d, *J*= 8.4 Hz, 1H), 7.46 (d, *J*= 8.1 Hz, 2H), 7.33 (d, *J*= 8.4 Hz, 1H), 7.25 (d, *J*= 8.0 Hz, 2H), 4.60 -4.34 (m, 2H), 2.87 (s, 3H). |
| 71 | 3-acetyl-8-bromo-5-chloro-2-((4-(trifluoromethoxy)benzyl)sulfinyl)q uinolin-4(1H)-one | 522.72 | 522 | ¹H NMR (300 MHz, CDCl₃)δ10.24(br,1H),7.6 9(d,*J*= 8.5 Hz, 1H), 7.30 (d, *J*= 8.4 Hz, 1H), 7.14 (d, *J*= 8.7 Hz, 2H), 7.04 (d, *J*= 8.1 Hz, 2H), 4.42 (q, *J*= 12.8 Hz, 2H), 2.84 (s, 3H). |
| 72 | 3-acetyl-8-bromo-5-chloro-2-(((5-(trifluoromethyl)furan-2-yl)methyl)sulfinyl)quinolin-4(1H)-one | 496.68 | 496 | ¹H NMR (300 MHz, CDCl₃)δ10.34 (s, 1H), 7.75 (d, *J*= 8.5 Hz, 1H), 7.36 (d, *J*= 8.4 Hz, 1H), 6.71 (s, 1H), 6.59 (d, *J*= 3.1 Hz, 1H), 4.58 (dd, *J=* 71.0, 14.1 Hz, 2H), 2.87 (s, 3H). |
| 73 | 4-(((3-acetyl-8-bromo-5-chloro-4-oxo-1,4-dihydroquinolin-2-yl)sulfinyl)methyl)benzonitrile | 463.73 | 463 | ¹H NMR (300 MHz, CDCl₃)δ10.21(s, 1H), 775-7.72(d, *J*=8.4 Hz, 1H), 7.52-7.50(d, *J*=8.04 Hz, 2H), 7.36-7.33(d, *J*=8.43 Hz, 1H), 7.27-7.25(d, *J*=8.1 Hz, 2H), 4.50-4.39(q, J=12.4, 6.33 Hz, 2H), 2.84(s, 3H). |
| 74 | 3-acetyl-8-bromo-5-chloro-2-((2-chloro-6-fluorobenzyl)sulfinyl)quinolin-4(1H)-one | 491.15 | 491 | ¹H NMR (300 MHz, CDCl₃)δ10.6(s,1H),7.75-7.72(d,J=8.4 Hz, 1H), 7.35-7.33(d, *J*=8.4 Hz, 1H), 7.31-7.28(m, 1H), 7.24-7.18(t, *J*=8.8 Hz, 1H), 7.01-6.96(t, *J*=8.0 Hz, 1H), 5.08-5.05(q, *J*=1.9, 11, 120 Hz, 2H), 2.82(s, 3H). |
| 75 | 3-acetyl-8-bromo-5-chloro-2-((2-methoxy-4-(pentafluoro-16-sulfanyl)benzyl)sulfinyl)quinolin-4(1H)-one | 594.79 | 594 | ¹H NMR (300 MHz, CDCl₃)δ10.26(s,1H),7.6 9-7.67(d,*J*=8.40 Hz, 1H), 7.31-7.28(d, *J*=8.43 Hz, 1H), 4.80-4.40(q, *J*=13.0, 95.7 Hz, 2H), 2.82(s, 3H). |
| 76 | 3-acetyl-8-bromo-5-chloro-2-((3-fluoro- 5 -(pentafluoro-16-sulfanyl)benzyl)sulfinyl)quinolin-4(1H)-one | 582.75 | 582 | ¹H NMR (300 MHz, CDCl₃)δ10.13 (s, 1H), 7.70 (d, *J*= 8.5 Hz, 1H), 7.38 (s, 1H), 7.33 (d, *J*= 8.6 Hz, 1H), 7.21 (d, *J*= 8.1 Hz, 1H), 7.09 (s, 1H), 4.44 (s, 2H), 2.84 (s, 3H). |
| 77 | 3-acetyl-8-bromo-5-chloro-2-((3-(pentafluoro-16-sulfanyl)benzyl)sulfinyl)quinolin-4(1H)-one | 564.76 | 564 | ¹H NMR (300 MHz, CDCl₃)δ10.06 (s, 1H), 7.59 -7.68 (m, 2H), 7.29 -7.38 (m, 4H), 4.47 (q, *J*= 12.8 Hz, 2H), 2.85 (s, 3H). |
| 78 | 3-acetyl-8-bromo-5-chloro-2-(((perfluorophenyl)methyl)sulfinyl)q uinolin-4(1H)-one | 528.67 | 528 | ¹H NMR (300 MHz, CDCl₃)δ10.48(s,1H),7.7 9-7.77(d,*J*=8.43 Hz,1H), 7.38-7.36(d, *J*=8.31 Hz, 1H), 4.49-4.40(q, *J*=13.0, 128 Hz, 2H), 2.83(s, 3H). |
| 79 | 3-acetyl-5,8-dichloro-2-((4-((trifluoromethyl)thio)benzyl)sulfiny 1)quinolin-4(1H)-one | 494.32 | 494 | ¹H NMR (300 MHz, CDCl₃)δ10.24(br s, 1H), 7.56-7.49 (m, 4H), 7.39-7.37(d,*J*=8.52 Hz, 2H), 7.21-7.18(d, *J*=7.68 Hz, 2H), 4.52-4.40(q, *J*=13.4 Hz, 2H), 2.87(s, 3H). |
| 80 | 3-acetyl-5,8-difluoro-2-((4-(pentafluoro-16-sulfanyl)benzyl)sulfinyl)quinolin-4(1H)-one | 487.40 | 487 | ¹H NMR (300 MHz, CDCl₃)δ9.78(br s, 1H), 7.59-7.58 (d, *J*=8.49 Hz, 1H), 7.37-7.22(m, 3H), 7.06-6.98(m, 1H), 4.41(s, 2H), 2.84(s, 3H). |
| 81 | 3-acetyl-5,8-difluoro-2-(((5-(trifluoromethyl)furan-2-yl)methyl)sulfinyl)quinolin-4(1H)-one | 419.32 | 419 | ¹H NMR (300 MHz, CDCl₃)δ9.93(br s, 1H), 7.41-7.33(m, 1H), 7.08-7.00 (m, 1H), 6.71-6.69(m, 1H), 6.56-6.55(m, 1H), 4.66(d, *J*=14.1 Hz, 1H), 4.48-4.44(d, *J*=14.07 Hz, 1H), 2.83(s, 3H). |
| 82 | 3-acetyl-5,8-difluoro-2-(((5-methylisoxazol-3-yl)methyl)sulfinyl)quinolin-4(1H)-one | 366.34 | 366 | ¹H NMR (300 MHz, CDCl₃)δ 10.45(br s, 1H), 7.61-7.59(d, *J*=8.49 Hz, 2H), 7.41-7.38 (d, *J*=8.46 Hz, 1H), 6.16(s, 1H), 4.51-4.39(q, *J*=13.5, 21.8 Hz, 2H), 2.81(s, 3H), 2.37(s, 3H). |
| 83 | 3-acetyl-5,8-dichloro-2-((4-iodobenzyl)sulfinyl)quinolin-4(1H)-one | 520.16 | 520 | ¹H NMR (300 MHz, CDCl₃)δ10.11(br s, 1H), 7.60-7.57(d, *J*= 8.46 Hz, 1H), 7.51-7.49 (d, J= 8.22 Hz, 2H), 7.40-7.37(d, *J*= 8.43 Hz, 1H), 6.83-6.81(d, *J*= 8.22 Hz, 2H), 4.32(s, 2H), 2.83(s, 3H). |
| 84 | 3-acetyl-8-bromo-5-chloro-2-((pyridin-3-ylmethyl)sulfinyl)quinolin-4(1H)-one | 439.71 | 439 | ¹H NMR (300 MHz, DMSO) δ8.41 (d, *J*= 4.6 Hz, 1H), 8.21 (s, 1H), 7.99 (d, *J*= 8.5 Hz, 1H), 7.52 (d, *J*= 7.9 Hz, 1H), 7.44 (d, *J*= 8.5 Hz, 1H), 7.25 (dd, *J*= 7.6, 4.6 Hz, 1H), 4.71 (d, *J*= 13.1 Hz, 1H), 4.37 (d, *J*= 13.0 Hz, 1H), 2.68 (s, 3H). |
| 85 | 5,8-difluoro-3 -isobutyryl-2-((4-((trifluoromethyl)thio)benzyl)sulfiny l)quinolin-4(1H)-one | 489.48 | 489 | ¹H NMR (300 MHz, CDCl₃+MeOD)δ7.60-6.92(m, 6H), 4.45-4.41(m, 1H), 4.35-4.31(m, 1H), 4.09-4.00 (m, 1H), 1.19-1.17(d, *J*= 6 Hz, 3H), 1.11-1.08(d, *J*= 7.17 Hz, 3H). |
| 86 | 5,8 -dichloro-3 -isobutyryl-2-(((5 - methylisoxazol-3 - yl)methyl)sulfinyl)quinolin-4(1H)-one | 427.30 | 427 | ¹H NMR (300 MHz, CDCl₃)δ10.43(br s, 1H), 7.59-7.56(d, *J*= 8.43 Hz, 1H), 7.38-7.35(d, *J*= 8.43 Hz, 1H), 6.13(s, 1H), 4.50-4.39(d, *J*= 8.54 Hz, 2H), 4.14-4.05(m, 1H), 2.34(s, 3H). 1.21-1.15(dd, *J*= 6.8, 11.6 Hz, 6H). |
| 87 | 3-benzoyl-5,8-difluoro-2-((4-(pentafluoro-16-sulfanyl)benzyl)sulfinyl)quinolin-4(1H)-one | 549.48 | 549 | ¹H NMR (300 MHz, CDCl₃)δ7.74-7.27(m, 10H), 7.00-6.92 (m, 1H), 4.80-4.76(d, *J*= 12.66 Hz, 1H), 4.53-4.49(d, *J*= 12.6 Hz, 1H). |
| 88 | 3-benzoyl-5,8-dichloro-2-(((5-methylisoxazol-3-yl)methyl)sulfinyl)quinolin-4(1H)-one | 461.31 | 461 | ¹H NMR (300 MHz, CDCl₃)δ7.77-7.75(d, *J*= 6.75 Hz, 2H), 7.74-7.70 (d, *J*= 8.28 Hz, 1H), 7.59-7.31(m, 5H), 6.18(s, 1H), 4.68(s, 2H), 3.10(s, 3H). |
| 89 | methyl 5-(((3-acetyl-5,8-dichloro-4-oxo-1,4-dihydroquinolin-2-yl)sulfinyl)methyl)furan-2-carboxylate | 442.26 | 442 | ¹H NMR (300 MHz, CDCl₃)δ10.24 (s, 1H), 7.56 (d, *J*= 8.5 Hz, 1H), 7.38 (d, *J*= 8.5 Hz, 1H), 7.05 (d, *J*= 3.4 Hz, 1H), 6.59 (d, *J*= 3.4 Hz, 1H), 4.65 (d, *J*= 13.9 Hz, 1H), 4.44 (d, *J*= 13.9 Hz, 1H), 3.60 (s, 3H), 2.85 (s, 3H). |
| 91 | methyl 4-(((3-acetyl-5,8-dichloro-4-oxo-1,4-dihydroquinolin-2-yl)sulfinyl)methyl)benzoate | 452.30 | 452 | ¹H NMR (300 MHz, CDCl₃)δ10.23 (s, 1H), 7.87 (d, *J*= 8.2 Hz, 2H), 7.52 (d, *J*= 8.4 Hz, 1H), 7.36 (d, *J*= 8.4 Hz, 1H), 7.23 (d, *J*= 8.2 Hz, 2H), 4.44 (q, *J*= 12.5 Hz, 2H), 3.89 (d, *J*= 5.4 Hz, 3H), 2.85 (s, 3H). |
| 93 | 3-acetyl-5-methoxy-2-((4-(pentafluoro-16-sulfanyl)benzyl)sulfinyl)quinolin-4(1H)-one | 481.45 | 481 | ¹H NMR (300 MHz, CDCl₃)δ9.63 (s, 1H), 8.28 (d, *J*= 9.0 Hz, 1H), 7.57 (d, *J*= 8.6 Hz, 2H), 7.23 (s, 1H), 7.04 (dd, *J*= 9.0, 2.2 Hz, 1H), 6.54 (d, *J*= 2.2 Hz, 1H), 4.39 (dd, *J*= 36.0, 12.5 Hz, 2H), 3.87 (d, *J*= 10.5 Hz, 3H), 2.87 (s, 3H). |
| 94 | 3-acetyl-5-methoxy-2-(((5-methylisoxazol-3-yl)methyl)sulfinyl)quinolin-4(1H)-one | 360.38 | 360 | ¹H NMR (300 MHz, CDCl₃)δ10.43 (s, 1H), 8.34 (d, *J*= 9.0 Hz, 1H), 7.08 (d, *J*= 8.9 Hz, 1H), 6.93 (s, 1H), 6.08 (s, 1H), 4.45 (dd, *J*= 60.6, 13.3 Hz, 2H), 3.91 (s, 3H), 2.87 (s,3H), 2.32 (s, 3H). |
| 95 | 8-bromo-5-chloro-3-isobutyryl-2-(((5-methylisoxazol-3-yl)methyl)sulfinyl)quinolin-4(1H)-one | 471.75 | 471 | ¹H NMR (300 MHz, CDCl₃)δ10.49(br s, 1H), 7.75-7.72 (d, *J*=8.43 Hz, 1H), 7.34-7.31(d, *J*=8.37 Hz, 1H), 6.13(s, 1H), 4.47(s, 2H), 4.16-4.07(m, 1H), 2.35(s, 3H), 1.23-1.17(dd, *J*=6.7, 12.0 Hz, 6H). |
| 96 | 8-bromo-5-chloro-3-(cyclopropanecarbonyl)-2-(((5-methylisoxazol-3-yl)methyl)sulfinyl)quinolin-4(1H)-one | 469.73 | 469 | ¹H NMR (300 MHz, CDCl₃)δ10.44(br s, 1H), 7.75-7.72 (d, *J*=8.43 Hz, 1H), 7.34-7.31(d, *J*=8.4 Hz, 1H), 6.11(s, 1H), 4.48-4.37(q, *J*=10.5 Hz, 2H), 3.70-3.62(m, 1H), 2.35(s, 3H), 1.28-1.05(m, 4H). |
| 97 | 5,8-dichloro-3-(cyclopropanecarbonyl)-2-(((5-methylisoxazol-3-yl)methyl)sulfinyl)quinolin-4(1H)-one | 425.28 | 425 | ¹H NMR (300 MHz, CDCl₃)δ10.42(br s, 1H), 7.61-7.58 (d, *J*=8.43 Hz, 1H), 7.41-7.38(d, *J*=8.43 Hz, 1H), 6.12(s, 1H), 4.49-4.37(q, *J*=11.7 Hz, 2H), 3.71-3.62(m, 1H), 2.36(s, 3H), 1.29-1.06(m, 4H). |
| 98 | 5-(((3-acetyl-8-bromo-5-chloro-4-oxo-1,4-dihydroquinolin-2-yl)sulfinyl)methyl)thiophene-2-carbonitrile | 469.75 | 469 | ¹H NMR (300 MHz, CDCl₃)δ10.31(br s, 1H), 7.79-7.77 (d, *J*= 8.43 Hz, 1H), 7.42-7.36(m, 2H), 6.91-6.86(d, *J*= 3.84 Hz, 1H), 4.77-4.72(d, *J*= 13.74, 1H), 4.63-4.59(d, *J*= 13.71, 1H), 2.86(s, 3H). |
| 99 | 2-(((6-(1H-pyrazol-1-yl)pyridin-3-yl)methyl)sulfinyl)-3-acetyl-8-bromo-5-chloroquinolin-4(1H)-one | 505.77 | 505 | ¹H NMR (300 MHz, DMSO-d₆)δ10.00(br s, 1H), 8.46-8.45 (s,1H), 8.11(s, 1H), 7.89-7.86(d, *J*=8.46 Hz, 1H), 7.80(s, 1H), 7.79-7.71(m, 2H), 7.41-7.38(d, *J*=8.37 Hz, 1H), 6.55(s, 1H), 4.79-4.75(d, *J*=12.99 Hz, 1H), 4.43-4.38(d, *J*=13.08 Hz, 1H), 2.70(s, 3H). |
| 100 | 3-acetyl-2-(((6-aminopyridin-3-yl)methyl)sulfinyl)-8-bromo-5-chloroquinolin-4(1H)-one | 454.72 | 454 | ¹H NMR (300 MHz, DMSO-d₆)δ8.22-7.65 (m, 4H), 7.09-7.06(m, 1H), 4.36-4.32(m,1H), 4.14-4.11(m, 1H), 2.88(s, 3H). |
| 101 | 8-bromo-5-chloro-3-(cyclopropanecarbonyl)-2-((4-((trifluoromethyl)thio)benzyl)sulfiny l)quinolin-4(1H)-one | 564.82 | 564 | ¹H NMR (300 MHz, DMSO-d₆)δ10.11(br s, 1H), 8.08-8.06 (d,*J*=8.43 Hz, 1H), 7.55-7.52(d, J =7.71 Hz, 2H), 7.45-7.43(d, *J*=8.37 Hz, 1H), 7.20-7.17(d, *J*=7.71 Hz, 2H), 4.65-4.61(d, *J*=12.66 Hz, 1H), 4.42-4.37(d, *J*=12.87 Hz, 1H), 3.55-3.47(m, 1H), 1.28-1.02(m, 4H). |
| 102 | 3-acetyl-8-bromo-5-chloro-2-(((2-methyl-6-(trifluoromethyl)pyridin-3-yl)methyl)sulfinyl)quinolin-4(1H)-one | 521.73 | 521 | ¹H NMR (300 MHz, DMSO-d₆)δ10.32(br s, 1H), 8.02-7.99(d, *J*=8.46 Hz, 1H), 7.68-7.65(d, *J*=7.98 Hz, 1H), 7.55-7.52(d, *J*=7.89 Hz, 1H), 7.45-7.42(d, *J*=8.49 Hz, 1H), 4.95-4.91(d, *J*=13.41 Hz, 1H), 4.51-4.46(d,J= 13.08 Hz, 2H), 2.68(s, 3H), 2.61(s, 3H). |
| 103 | N-(4-(((3-acetyl-8-bromo-5-chloro-4-oxo-1,4-dihydroquinolin-2-yl)sulfinyl)methyl)phenyl)methanes ulfonamide | 531.82 | 531 | ¹H NMR (300 MHz, DMSO-d₆)δ10.25(br s, 1H), 8.01 (m, 1H), 7.67-7.64(m, 1H), 7.41-6.98(m, 6H), 4.79-4.50(m, 2H), 4.30-4.22(m, 2H), 2.81(s, 3H), 2.68(s, 3H). |
| 104 | 3-acetyl-8-bromo-5-chloro-2-(((6-chloropyridin-3-yl)methyl)sulfinyl)quinolin-4(1H)-one | 474.15 | 474 | ¹H NMR (300 MHz, CDCl₃)δ10.20 (s, 1H), 8.10 (s, 1H), 7.76 (d, *J*= 8.4 Hz, 1H), 7.51 (d, *J*= 7.8 Hz, 1H), 7.36 (d, *J*= 8.4 Hz, 1H), 7.24 (d, *J*= 8.1 Hz, 1H), 4.41 (dd, *J*= 26.8, 13.0 Hz, 2H), 2.86 (s, 3H). |
| 105 | 3-acetyl-8-bromo-5-chloro-2-(((6-((2-methoxyethyl)amino)pyridin-3-yl)methyl)sulfinyl)quinolin-4(1H)-one | 512.80 | 512 | ¹H NMR (300 MHz, CDC1₃) δ 12.59 (s,1H), 7.75 (d,*J*= 8.2 Hz, 1H), 7.63 (d, *J*= 7.6 Hz, 1H), 7.04 (d, *J*= 8.2 Hz, 2H), 6.11 (d, *J*= 7.6 Hz, 1H), 4.61 ― 4.53 (m, 1H), 3.90 ― 3.84 (m, 1H), 3.71 ― 3.65 (m, 2H), 3.64 ― 3.55 (m, 2H), 3.41 (s, 3H), 3.33 (s, 3H). |
| 106 | 3-acetyl-8-bromo-5-chloro-2-(((4-methyl- 1,2,5 -oxadiazol-3 - yl)methyl)sulfinyl)quinolin-4(1H)-one | 444.68 | 444 | ¹H NMR (300 MHz, DMSO-d₆)δ 7.76-7.73(d, *J*= 8.5 Hz, 1H), 7.08-7.06(d, *J*= 8.5 Hz, 1H), 4.52-4.48(d, *J*= 12.0 Hz, 1H), 4.28-4.23 (d, *J*= 12.0 Hz, 1H), 2.50(s, 3H), 2.44(s, 3H). |
| 107 | 2-(((1H-pyrrolo[2,3-b]pyridin-5-yl)methyl)sulfinyl)-3-acetyl-8-bromo-5-chloroquinolin-4(1H)-one | 478.75 | 478 | ¹H NMR (300 MHz, DMSO-d₆)δ11.62(br s, 1H), 8.00-7.32 (m, 5H), 6.38(s, 1H), 4.62-4.58(m, 1H), 4.20-4.10(m, 1H), 2.60(s, 3H). |

### Evaluation of compounds of the present disclosure

### Evaluation of inhibitory effect of compounds on DNA binding of c-Myc/Max

### 1. Protein assay

### 1) Preparation of recombinant c-Myc and Max proteins

Recombinant proteins were prepared as described in the following references: K. C. Jung et al., Fatty Acids, Inhibitors for the DNA Binding of c-Myc/Max Dimer, Suppress Proliferation and Induce Apoptosis of Differentiated HL-60 Human Leukemia Cell, Leukemia, 2006, 20(1), 122-7 or Kyung-Chae Jeong et al., Small-Molecule Inhibitors of c-Myc Transcriptional Factor Suppress Proliferation and Induce Apoptosis of Promyelocytic Leukemia Cell via Cell Cycle Arrest, Mol. BioSyst., 2010, 6, 1503-1509.

### 2) Electrophoretic mobility shift assay (EMSA)

The inhibitory activity of each candidate compound on DNA binding of recombinant c-Myc/Max was measured using an electrophoretic mobility shift assay (EMSA). The ratio of protein-DNA complexes in each sample was evaluated by measuring band intensity. The oligonucleotides (E-box, 5'-biotin-GGAAGCAGACCACGTGGTCTGCTTCC-3'-biotin) corresponding to the consensus binding site of Myc/Max were dimerized through an annealing process. The protein mixture was incubated at room temperature for 5 minutes, and a DMSO solution containing each candidate compound was added thereto. The mixture was further incubated for 5 minutes, and the biotinylated DNA was added. To achieve a state of equilibrium, the final mixture was incubated at room temperature for 10 minutes. The protein-DNA complexes were separated from unbound free DNA by pre-electrophoresis using 8% polyacrylamide gel and 1× TBE buffer. After pre-electrophoresis, electrophoresis was performed at 120 V for 1 hour in 1×TBE buffer. Each band was visualized using HRP-conjugated streptavidin and an ECL solution, and band intensity was measured using image analysis software.

### 2. Cell based assay

Commercially available bladder cancer cell lines were treated with trypsin-EDTA and seeded in each well of a 96 well plate, followed by incubation for 24 hours. After incubation, candidate compounds were added to each well at a final concentration of 0 to 2 µM. The compound-treated cells were further incubated for 72 hours. Cell viability was measured using an ATP detection method (CellTiter-Glo Luminescent Cell Viability Assay, Promega).

IC₅₀ values calculated from an *in vitro* assay and a proliferation assay using compounds according to the present disclosure are summarized in Table 5 below.

**[Table 5]**

| | | | | | |
|---|---|---|---|---|---|
| | protein | cell based assay | | | |

| Compound Number | assay | | | | |
|---|---|---|---|---|---|
| | | cell line 1 MBT-2 | cell line 2 KU19-19 | cell line 3 253J | cell line 4 UM-UC-3 |
| 4 | < 1 µM | 0.96 µM | 1.05 µM | 1.00 µM | 1.33 µM |
| 11 | < 1 µM | > 2 µM | > 2 µM | 1.41 µM | 1.49 µM |
| 13 | < 1 µM | > 2 µM | > 2 µM | > 2 µM | > 2 µM |
| 15 | < 1 µM | > 2 µM | 1.91 µM | > 2 µM | > 2 µM |
| 17 | < 1 µM | > 2 µM | > 2 µM | > 2 µM | > 2 µM |
| 26 | < 1 µM | > 2 µM | > 2 µM | > 2 µM | > 2 µM |
| 28 | < 1 µM | 0.79 µM | 1.46 µM | 0.74 µM | 0.79 µM |
| 29 | < 1 µM | 1.20 µM | 1.68 µM | 0.96 µM | 1.13 µM |
| 31 | < 1 µM | 1.28 µM | 1.45 µM | 0.89 µM | 1.02 µM |
| 33 | < 1 µM | 0.90 µM | 1.42 µM | 1.39 µM | 1.11 µM |
| 34 | < 1 µM | > 2 µM | > 2 µM | > 2 µM | > 2 µM |
| 44 | < 1 µM | 1.15 µM | 1.27 µM | 1.24 µM | 0.97 µM |
| 46 | < 1 µM | 1.84 µM | 1.80 µM | 1.96 µM | 1.22 µM |
| 48 | < 1 µM | > 2 µM | > 2 µM | > 2 µM | 1.82 µM |
| 50 | < 1 µM | 1.58 µM | 1.69 µM | 1.43 µM | 1.26 µM |
| 51 | < 1 µM | 0.76 µM | 1.26 µM | 1.13 µM | 0.63 µM |
| 53 | < 1 µM | > 2 µM | > 2 µM | > 2 µM | > 2 µM |
| 54 | < 1 µM | > 2 µM | > 2 µM | > 2 µM | > 2 µM |
| 55 | < 1 µM | > 2 µM | 1.38 µM | 1.33 µM | 1.58 µM |
| 56 | < 1 µM | 1.18 µM | 0.94 µM | 1.11 µM | 1.25 µM |
| 57 | < 1 µM | > 2 µM | 1.83 µM | 1.92 µM | > 2 µM |
| 58 | < 1 µM | 0.86 µM | 0.92 µM | 0.92 µM | 1.22 µM |
| 59 | < 1 µM | 1.30 µM | 1.23 µM | 1.14 µM | 1.37 µM |
| 60 | < 1 µM | > 2 µM | 1.56 µM | 1.71 µM | > 2 µM |
| 63 | < 1 µM | > 2 µM | > 2 µM | > 2 µM | > 2 µM |
| 64 | < 1 µM | 0.84 µM | 1.41 µM | 0.95 µM | 1.06 µM |
| 68 | < 1 µM | 1.07 µM | 1.25 µM | 0.97 µM | 1.11 µM |
| 69 | < 1 µM | 1.26 µM | 1.50 µM | 1.08 µM | 1.20 µM |
| 70 | < 1 µM | 1.33 µM | 1.75 µM | 1.04 µM | 1.26 µM |
| 71 | < 1 µM | 1.47 µM | 1.60 µM | 1.11 µM | 1.61 µM |
| 72 | < 1 µM | 1.01 µM | 1.37 µM | 0.83 µM | 0.82 µM |
| 73 | < 1 µM | 1.00 µM | 2.00 µM | 1.34 µM | 1.62 µM |
| 74 | < 1 µM | 1.15 µM | 1.64 µM | 0.81 µM | 1.54 µM |
| 75 | < 1 µM | 1.05 µM | 1.21 µM | 0.86 µM | 1.01 µM |
| 76 | < 1 µM | 1.86 µM | 1.86 µM | 1.24 µM | 1.39 µM |
| 77 | < 1 µM | 1.45 µM | 1.38 µM | 0.90 µM | 1.18 µM |
| 78 | < 1 µM | 1.53 µM | > 2 µM | 1.29 µM | 1.39 µM |
| 79 | < 1 µM | > 2 µM | > 2 µM | > 2 µM | > 2 µM |
| 80 | < 1 µM | > 2 µM | > 2 µM | > 2 µM | > 2 µM |
| 81 | < 1 µM | > 2 µM | > 2 µM | > 2 µM | > 2 µM |
| 82 | < 1 µM | > 2 µM | > 2 µM | > 2 µM | > 2 µM |
| 83 | < 1 µM | 1.35 µM | 1.60 µM | 1.42 µM | 1.31 µM |
| 84 | < 1 µM | 0.67 µM | 1.50 µM | 0.87 µM | 0.76 µM |
| 85 | < 1 µM | > 2 µM | > 2 µM | > 2 µM | > 2 µM |
| 86 | < 1 µM | 0.81 µM | 1.41 µM | 1.06 µM | 1.26 µM |
| 87 | < 1 µM | > 2 µM | > 2 µM | > 2 µM | > 2 µM |
| 88 | < 1 µM | > 2 µM | > 2 µM | > 2 µM | > 2 µM |
| 89 | < 1 µM | 1.27 µM | > 2 µM | 1.84 µM | 1.47 µM |
| 91 | < 1 µM | 1.37 µM | > 2 µM | > 2 µM | 1.56 µM |
| 93 | < 1 µM | > 2 µM | > 2 µM | > 2 µM | > 2 µM |
| 94 | < 1 µM | 1.53 µM | > 2 µM | > 2 µM | > 2 µM |
| 95 | < 1 µM | 0.80 µM | 1.29 µM | 0.77 µM | 1.06 µM |
| 96 | < 1 µM | 1.06 µM | > 2 µM | 1.49 µM | 1.63 µM |
| 97 | < 1 µM | 0.81 µM | 1.62 µM | 1.20 µM | 1.27 µM |
| 98 | < 1 µM | 0.84 µM | 1.90 µM | 1.38 µM | 1.52 µM |
| 99 | < 1 µM | 0.47 µM | 1.12 µM | 0.80 µM | 0.77 µM |
| 100 | < 1 µM | > 2 µM | > 2 µM | > 2 µM | > 2 µM |
| 101 | < 1 µM | > 2 µM | > 2 µM | 1.82 µM | 1.93 µM |
| 102 | < 1 µM | 0.77 µM | 1.58 µM | 0.83 µM | 1.07 µM |
| 103 | < 1 µM | > 2 µM | > 2 µM | > 2 µM | > 2 µM |
| 104 | < 1 µM | 0.71 µM | 1.50 µM | 0.87 µM | 1.13 µM |
| 105 | < 1 µM | > 2 µM | > 2 µM | > 2 µM | > 2 µM |
| 106 | < 1 µM | > 2 µM | > 2 µM | > 2 µM | > 2 µM |
| 107 | < 1 µM | > 2 µM | > 2 µM | > 2 µM | > 2 µM |

As shown in Table 5, the compounds according to the present disclosure were highly effective in inhibiting c-Myc/Max/DNA complex formation, and were particularly effective in suppressing bladder cancer cell lines.

### Evaluation of selectivity of compounds of the present disclosure

The selectivity of the compounds of the present disclosure to cancer cells was evaluated in the same manner as described in "2. Cell based assay". As a comparative example, KSI-3716 compound of Formula 4, which is a known compound, was used. Measurement results are summarized in Table 6 below.

**[Table 6]**

| Compound | Cytotoxicity (µM) | | | | |
|---|---|---|---|---|---|
| Compd. | MBT-2 | KU19-19 | UM-UC-3 | 253J | **RT4** |
| KSI-3716 | 1.0 | 0.4 | 0.9 | 1.2 | **1.5** |
| Compound 4 | 1.0 | 1.1 | 1.5 | 1.1 | **> 10** |
| Compound 33 | 0.9 | 1.4 | 1.6 | 1.6 | **> 10** |

| | | | | | |
|---|---|---|---|---|---|
| 253J: human urinary tract transitional cell carcinoma UM-UC-3: human urinary bladder transitional cell carcinoma RT4: human urinary bladder transitional cell papilloma | | | | | |

As shown in Table 6, compound KSI-3716 causes nonselective cell death in both benign (RT4) and malignant (253J and UM-UC-3) bladder cancer cell lines, but the compounds of the present disclosure kill only malignant cancer cells with high selectivity.

The present disclosure provides novel compounds that can have various pharmacological activities by inhibiting c-Myc/Max/DNA complex formation. The compounds according to the present disclosure or pharmaceutically acceptable salts thereof are excellent in safety and has high selectivity in terms of inhibition of c-Myc/Max/DNA complex formation. Accordingly, various excellent effects can be exhibited.

When introducing elements of the present disclosure or the preferred embodiments thereof, the articles "a", "an", "the" and "said" are intended to mean that there are one or more of the elements. The terms "comprising," "including," and "having" are intended to be inclusive and mean that there may be additional elements other than the listed elements. Although the present disclosure is described with respect to particular aspects, it should not be construed as limiting the details of these aspects.

### [industrial applicability]

The present invention can provide a novel compound capable of inhibiting c-Myc / Max / DNA complex formation and exhibiting various pharmacological activities. The compound according to the present invention or a pharmaceutically acceptable salt thereof is excellent in safety and has high selectivity in terms of inhibition of c-Myc / Max / DNA complex formation, and thus can exhibit various excellent effects.

## Claims

1. A compound of Formula 3a or 3b or pharmaceutically acceptable salts thereof: 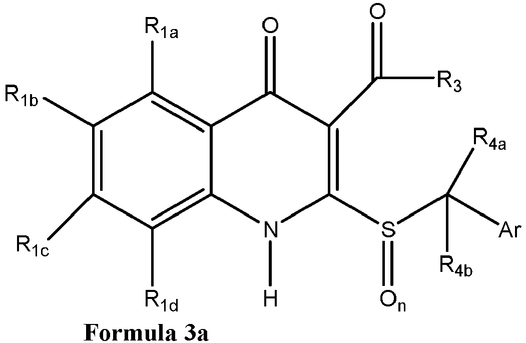 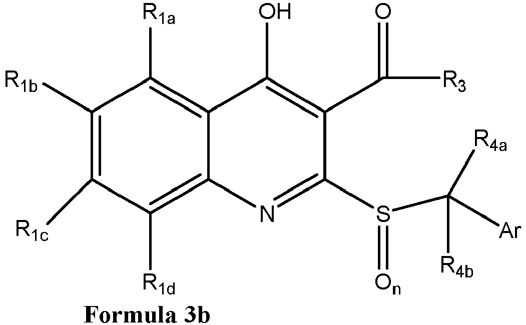 wherein
R₁ₐ to R_{1d} are each independently hydrogen, a halogen, C₁₋₆ alkyl, C₁₋₆ haloalkyl, C₁₋₆ hydroxyalkyl, C₁₋₆ alkoxy, C₁₋₆ haloalkoxy, C₂₋₁₀ alkenyl, C₂₋₁₀ haloalkenyl, C₂₋₁₀ alkynyl, C₂₋₁₀ haloalkynyl, a hydroxyl group, nitro, cyano, C₁₋₆ alkoxycarbonyl, amino, C₁₋₆ alkylamino, di(C₁₋₆ alkyl)amino, amino(C₁₋₆)alkyl, (C₁₋₆)alkylamino(C₁₋₆)alkyl, C₁₋₆ alkanoyl, C₃₋₇ cycloalkyl, an aryl, a heterocycle, or a heteroaryl, wherein R₁ₐ to R_{1d} are each independently unsubstituted or optionally substituted;
R₃ is C₁₋₄ alkyl, isoalkyl, cycloalkyl, phenyl, or C₁₋₄ haloalkyl;
n is 1 or 2;
R₄ₐ and R_{4b} are each independently hydrogen, a halogen, C₁₋₄ alkyl, C₁₋₄ haloalkyl, or C₁₋₄ alkyl in which one or more hydrogens are substituted with substituents other than halogen;
Ar is phenyl, heteroaryl being 5-6-membered and having heteroatoms selected independently from N, S, or O, or biheteroaryl being 8-12-membered and having heteroatoms selected independently from N, S, or O,
wherein Ar may be unsubstituted or may be optionally substituted with one or more of a halogen, C₁₋₆ alkyl, C₁₋₆ alkylthio, C₁₋₆ haloalkyl, C₁₋₆ haloalkylthio, C₁₋₆ alkoxy, C₁₋₆ haloalkoxy, C₂₋₁₀ alkenyl, C₂₋₁₀ haloalkenyl, C₂₋₁₀ alkynyl, C₂₋₁₀ haloalkynyl, a hydroxyl group, COOH, nitro, cyano, C₁₋₆ alkoxycarbonyl, amino, C₁₋₆ alkylamino, di(C₁₋₆ alkyl)amino, amino(C₁₋₆)alkyl, (C₁₋₆)alkylamino(C₁₋₆)alkyl, (C₁₋₆)alkoxy(C₁₋₆)alkylamino, (C₁₋₆)alkylamino(C₁₋₆) alkylamino, C₁₋₆ alkanoyl, SF₅, S(O)CF₃, SCF₃, NHC(=O)CH₃, C(=O)NHCH₃, NHSO2CH3, C₃₋₇ cycloalkyl, an aryl, benzoyl, a heterocycle, a heteroaryl, phenyl, oxazole, pyrazole, pyrrole, imidazole, thiazole, thiophene, pyridine, pyrimidine, furan, indole, benzopyrazole, benzothiazole, benzooxazole, isoxazole, benzoimidazole, or benzothiophene, and
wherein the substituents of Ar may be unsubstituted or may be optionally substituted with one or more of CF₃, a halogen, (C₁₋₃)alkyl, (C₁₋₃)haloalkyl, hydrogen, COOH, nitro, cyano, amino, di(C₁₋₃ alkyl)amino, NHC(=O)CH₃, or C(=O)NHCH₃.

2. The compounds according to claim 1, wherein any one or more of R₁ₐ to R_{1d} are halogens, R₃ is C₁₋₄ alkyl, isoalkyl, cycloalkyl, phenyl, or C₁₋₄ haloalkyl, and R₄ₐ and R_{4b} are C₁₋₄ alkyl, a halogen, or C₁₋₄ cycloalkyl.

3. A compound according to claim 1 selected from the group consisting of:
3-acetyl-2-(benzylsulfinyl)-8-bromo-5-chloroquinolin-4(1H)-one,
3-acetyl-8-bromo-5-chloro-2-((4-chlorobenzyl)sulfinyl)quinolin-4(1H)-one,
3-acetyl-8-bromo-5-chloro-2-(phenylsulfinyl)quinolin-4(1H)-one,
3-acetyl-8-bromo-5-chloro-2-((2-methoxyphenyl)sulfinyl)quinolin-4(1H)-one,
3-acetyl-8-bromo-2-((4-bromophenyl)sulfinyl)-5-chloroquinolin-4(1H)-one,
3-acetyl-8-bromo-5-chloro-2-((1-phenylethyl)sulfinyl)quinolin-4(1H)-one,
3-(((3-acetyl-8-bromo-5-chloro-4-oxo-1,4-dihydroquinolin-2-yl)sulfinyl)methyl)benzonitrile,
3-acetyl-8-bromo-5-chloro-2-((2,4-difluorobenzyl)sulfinyl)quinolin-4(1H)-one,
3-acetyl-8-bromo-5-chloro-2-((3-chloro-4-fluorobenzyl)sulfinyl)quinolin-4(1H)-one,
3-acetyl-8-bromo-5-chloro-2-((4-nitrobenzyl)sulfinyl)quinolin-4(1H)-one,
3-acetyl-2-(benzylsulfonyl)-8-bromo-5-chloroquinolin-4(1H)-one,
3-acetyl-8-bromo-5-chloro-2-((2,5-dichlorobenzyl)sulfinyl)quinolin-4(1H)-one,
3-acetyl-8-bromo-5-chloro-2-((3,5-difluorobenzyl)sulfinyl)quinolin-4(1H)-one,
3-acetyl-8-bromo-5-chloro-2-((3-iodobenzyl)sulfinyl)quinolin-4(1H)-one,
3-acetyl-8-bromo-5-chloro-2-((3-fluorobenzyl)sulfinyl)quinolin-4(1H)-one,
3-acetyl-8-bromo-5-chloro-2-(((5-methylisoxazol-3-yl)methyl)sulfinyl)quinolin-4(1H)-one,
1-(2-(benzylsulfinyl)-8-bromo-5-chloro-4-hydroxyquinolin-3-yl)ethan-1-one,
1-(2-(benzylsulfonyl)-8-bromo-5-chloro-4-hydroxyquinolin-3-yl)ethan-1-one,
3-acetyl-8-bromo-5-chloro-2-((3-methoxybenzyl)sulfinyl)quinolin-4(1H)-one,
3-acetyl-8-bromo-5-chloro-2-((4-((trifluoromethyl)thio)benzyl)sulfinyl)quinolin-4(1H)-one,
3-acetyl-5,8-dichloro-2-((4-nitrobenzyl)sulfinyl)quinolin-4(1H)-one,
2-(((3-acetyl-8-bromo-5-chloro-4-oxo-1,4-dihydroquinolin-2-yl)sulfinyl)methyl)benzonitrile,
3-acetyl-8-bromo-5-chloro-2-((3,5-dimethoxybenzyl)sulfinyl)quinolin-4(1H)-one,
3-acetyl-8-bromo-2-((4-(tert-butyl)benzyl)sulfinyl)-5-chloroquinolin-4(1H)-one,
3-acetyl-2-((4-benzoylbenzyl)sulfinyl)-8-bromo-5-chloroquinolin-4(1H)-one,
3-acetyl-8-bromo-5-chloro-2-((4-((trifluoromethyl)sulfinyl)benzyl)sulfinyl)quinolin-4(1H)-one,
3-acetyl-8-bromo-5-chloro-2-((4-(pentafluoro-16-sulfanyl)benzyl)sulfinyl)quinolin-4(1H)-one,
3-acetyl-8-bromo-5-chloro-2-((2-fluoro-4-(pentafluoro-16-sulfanyl)benzyl)sulfinyl)quinolin-4(1H)-one,
3-acetyl-8-bromo-5-chloro-2-((4-(trifluoromethyl)benzyl)sulfinyl)quinolin-4(1H)-one,
3-acetyl-8-bromo-5-chloro-2-((4-(trifluoromethoxy)benzyl)sulfinyl)quinolin-4(1H)-one,
3-acetyl-8-bromo-5-chloro-2-(((5-(trifluoromethyl)furan-2-yl)methyl)sulfinyl)quinolin-4(1H)-one,
4-(((3-acetyl-8-bromo-5-chloro-4-oxo-1,4-dihydroquinolin-2-yl)sulfinyl)methyl)benzonitrile,
3-acetyl-8-bromo-5-chloro-2-((2-chloro-6-fluorobenzyl)sulfinyl)quinolin-4(1H)-one,
3-acetyl-8-bromo-5-chloro-2-((2-methoxy-4-(pentafluoro-16-sulfanyl)benzyl)sulfinyl)quinolin-4(1H)-one,
3-acetyl-8-bromo-5-chloro-2-((3-fluoro-5-(pentafluoro-16-sulfanyl)benzyl)sulfinyl)quinolin-4(1H)-one,
3-acetyl-8-bromo-5-chloro-2-((3-(pentafluoro-16-sulfanyl)benzyl)sulfinyl)quinolin-4(1H)-one,
3-acetyl-8-bromo-5-chloro-2-(((perfluorophenyl)methyl)sulfinyl)quinolin-4(1H)-one,
3-acetyl-5,8-dichloro-2-((4-((trifluoromethyl)thio)benzyl)sulfinyl)quinolin-4(1H)-one,
3-acetyl-5,8-difluoro-2-((4-(pentafluoro-16-sulfanyl)benzyl)sulfinyl)quinolin-4(1H)-one,
3-acetyl-5,8-difluoro-2-(((5-(trifluoromethyl)furan-2-yl)methyl)sulfinyl)quinolin-4(1H)-one,
3-acetyl-5,8-difluoro-2-(((5-methylisoxazol-3-yl)methyl)sulfinyl)quinolin-4(1H)-one,
3-acetyl-5,8-dichloro-2-((4-iodobenzyl)sulfinyl)quinolin-4(1H)-one,
3-acetyl-8-bromo-5-chloro-2-((pyridin-3-ylmethyl)sulfinyl)quinolin-4(1H)-one,
5,8-difluoro-3-isobutyryl-2-((4-((trifluoromethyl)thio)benzyl)sulfinyl)quinolin-4(1H)-one,
5,8-dichloro-3-isobutyryl-2-(((5-methylisoxazol-3-yl)methyl)sulfinyl)quinolin-4(1H)-one,
3-benzoyl-5,8-difluoro-2-((4-(pentafluoro-16-sulfanyl)benzyl)sulfinyl)quinolin-4(1H)-one,
3-benzoyl-5,8-dichloro-2-(((5-methylisoxazol-3-yl)methyl)sulfinyl)quinolin-4(1H)-one,
methyl 5-(((3-acetyl-5,8-dichloro-4-oxo-1,4-dihydroquinolin-2-yl)sulfinyl)methyl)furan-2-carboxylate,
methyl 4-(((3-acetyl-5,8-dichloro-4-oxo-1,4-dihydroquinolin-2-yl)sulfinyl)methyl)benzoate,
3-acetyl-5-methoxy-2-((4-(pentafluoro-16-sulfanyl)benzyl)sulfinyl)quinolin-4(1H)-one,
3-acetyl-5-methoxy-2-(((5-methylisoxazol-3-yl)methyl)sulfinyl)quinolin-4(1H)-one,
8-bromo-5-chloro-3-isobutyryl-2-(((5-methylisoxazol-3-yl)methyl)sulfinyl)quinolin-4(1H)-one,
8-bromo-5-chloro-3-(cyclopropanecarbonyl)-2-(((5-methylisoxazol-3-yl)methyl)sulfinyl)quinolin-4(1H)-one,
5,8-dichloro-3-(cyclopropanecarbonyl)-2-(((5-methylisoxazol-3-yl)methyl)sulfinyl)quinolin-4(1H)-one,
5-(((3-acetyl-8-bromo-5-chloro-4-oxo-1,4-dihydroquinolin-2-yl)sulfinyl)methyl)thiophene-2-carbonitrile,
2-(((6-(1H-pyrazol-1-yl)pyridin-3-yl)methyl)sulfinyl)-3-acetyl-8-bromo-5-chloroquinolin-4(1H)-one,
3-acetyl-2-(((6-aminopyridin-3-yl)methyl)sulfinyl)-8-bromo-5-chloroquinolin-4(1H)-one,
8-bromo-5-chloro-3-(cyclopropanecarbonyl)-2-((4-((trifluoromethyl)thio)benzyl)sulfinyl)quinolin-4(1H)-one,
3-acetyl-8-bromo-5-chloro-2-(((2-methyl-6-(trifluoromethyl)pyridin-3-yl)methyl)sulfinyl)quinolin-4(1H)-one,
N-(4-(((3-acetyl-8-bromo-5-chloro-4-oxo-1,4-dihydroquinolin-2-yl)sulfinyl)methyl)phenyl)methanesulfonamide,
3-acetyl-8-bromo-5-chloro-2-(((6-chloropyridin-3-yl)methyl)sulfinyl)quinolin-4(1H)-one,
3-acetyl-8-bromo-5-chloro-2-(((6-((2-methoxyethyl)amino)pyridin-3-yl)methyl)sulfinyl)quinolin-4(1H)-one,
3-acetyl-8-bromo-5-chloro-2-(((4-methyl-1,2,5-oxadiazol-3-yl)methyl)sulfinyl)quinolin-4(1H)-one,
2-(((1H-pyrrolo[2,3-b]pyridin-5-yl)methyl)sulfinyl)-3-acetyl-8-bromo-5-chloroquinolin-4(1H)-one,
or pharmaceutically acceptable salts thereof.

4. A composition comprising compounds according to any one claim of claims 1 to 3 or pharmaceutically acceptable salts thereof, and a pharmaceutically acceptable carrier.

5. A compound according to any one of claims 1-3, or the composition according to claim 4, for use in a method for treatment or prevention of cancer.

## Patentansprüche

1. Verbindung von Formel 3a oder 3b oder pharmazeutisch akzeptable Salze davon: 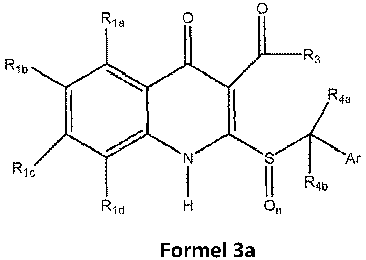 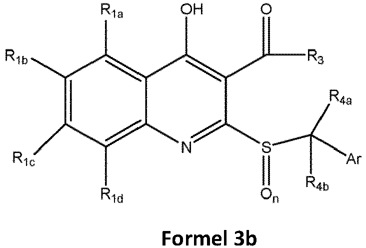 wobei
R₁ₐ bis R_{1d} jeweils unabhängig Wasserstoff, ein Halogen, C_{1―6}-Alkyl, C_{1―6}-Haloalkyl, C_{1―6}-Hydroxyalkyl, C_{1―6}-Alkoxy, C_{1―6}-Haloalkoxy, C_{2―10}-Alkenyl, C_{2―10}-Haloalkenyl, C_{2―10}-Alkynyl, C_{2―10}-Haloalkynyl, eine Hydroxylgruppe, Nitro, Cyano, C_{1―6}-Alkoxycarbonyl, Amino, C_{1―6}-Alkylamino, Di(C_{1―6}-alkyl)amino, Amino(C_{1―6})alkyl, (C_{1―6})Alkylamino(C_{1―6})alkyl, C_{1―6}-Alkanoyl, C_{3―7}-Cycloalkyl, ein Aryl, ein Heterozyklus oder ein Heteroaryl ist, wobei R₁ₐ bis R_{1d} jeweils unabhängig unsubstituiert oder wahlweise substituiert sind;
R₃ C_{1―4}-Alkyl, Isoalkyl, Cycloalkyl, Phenyl oder C_{1―4}-Haloalkyl ist;
n 1 oder 2 ist;
R₄ₐ und R_{4b} jeweils unabhängig Wasserstoff, ein Halogen, C_{1―4}-Alkyl, C_{1―4}-Haloalkyl oder C_{1―4}-Alkyl sind, bei dem ein oder mehrere Wasserstoffe mit anderen Substituenten als Halogen substituiert sind;
Ar Phenyl, Heteroaryl, das 5- bis 6-gliedrig ist und Heteroatome aufweist, die unabhängig aus N, S oder O ausgewählt sind, oder Biheteroaryl ist, das 8- bis 12-gliedrig ist und Heteroatome aufweist, die unabhängig aus N, S oder O ausgewählt sind,
wobei Ar unsubstituiert oder wahlweise mit einem oder mehreren von Halogen, C_{1―6}-Alkyl, C_{1―6}-Alkylthio, C_{1―6}-Haloalkyl, C_{1―6}-Haloalkylthio, C_{1―6}-Alkoxy, C_{1―6}-Haloalkoxy, C_{2―10}-Alkenyl, C_{2―10}-Haloalkenyl, C_{2―10}-Alkynyl, C_{2―10}-Haloalkynyl, einer Hydroxylgruppe, COOH, Nitro, Cyano, C_{1―6}-Alkoxycarbonyl, Amino, C_{1―6}-Alkylamino, Di(C_{1―6}-alkyl)amino, Amino(C_{1―6}-)alkyl, (C_{1―6})Alkylamino(C_{1―6})alkyl, (C_{1―6})Alkoxy(C_{1―6})alkylamino, (C_{1―6})Alkylamino(C_{1― 6})alkylamino, C_{1―6}-Alkanoyl, SF₅, S(O)CF₃, SCF₃, NHC(=O)CH₃, C(=O)NHCH₃, NHSO₂CH₃, C_{3―7}-Cycloalkyl, einem Aryl, Benzoyl, einem Heterozyklus, einem Heteroaryl, Phenyl, Oxazol, Pyrazol, Pyrrol, Imidazol, Thiazol, Thiophen, Pyridin, Pyrimidin, Furan, Indol, Benzopyrazol, Benzothiazol, Benzoxazol, Isoxazol, Benzimidazol oder Benzothiophen substituiert sein kann, und
wobei die Substituenten von Ar unsubstituiert oder wahlweise mit einem oder mehreren von CF₃, einem Halogen, (C_{1―3})Alkyl, (C_{1―3})Haloalkyl, Wasserstoff, COOH, Nitro, Cyano, Amino, Di(C_{1―3}-alkyl)amino, NHC(=O)CH₃ oder C(=O)NHCH₃ substituiert sein können.

2. Verbindungen nach Anspruch 1, wobei ein oder mehrere beliebige von R₁ₐ bis R_{1d} Halogene sind, R₃ C_{1―4}-Alkyl, Isoalkyl, Cycloalkyl, Phenyl oder C_{1―4}-Haloalkyl ist und R₄ₐ und R_{4b} C_{1―4}-Alkyl, ein Halogen oder C_{1―4}-Cycloalkyl sind.

3. Verbindung nach Anspruch 1, ausgewählt aus der Gruppe, bestehend aus:
3-Acetyl-2-(benzylsulfinyl)-8-brom-5-chlorchinolin-4(1H)-on,
3-Acetyl-8-brom-5-chlor-2-((4-chlorbenzyl)sulfinyl)chinolin-4(1H)-on,
3-Acetyl-8-brom-5-chlor-2-(phenylsulfinyl)chinolin-4(1H)-on,
3-Acetyl-8-brom-5-chlor-2-((2-methoxyphenyl)sulfinyl)chinolin-4(1H)-on,
3-Acetyl-8-brom-2-((4-bromphenyl)sulfinyl)-5-chlorchinolin-4(1H)-on,
3-Acetyl-8-brom-5-chlor-2-((1-phenylethyl)sulfinyl)chinolin-4(1H)-on,
3-(((3-Acetyl-8-brom-5-chlor-4-oxo-1,4-dihydrochinolin-2-yl)sulfinyl)methyl)benzonitril,
3-Acetyl-8-brom-5-chlor-2-((2,4-difluorbenzyl)sulfinyl)chinolin-4(1H)-on,
3-Acetyl-8-brom-5-chlor-2-((3-chlor-4-fluorbenzyl)sulfinyl)chinolin-4(1H)-on,
3-Acetyl-8-brom-5-chlor-2-((4-nitrobenzyl)sulfinyl)chinolin-4(1H)-on,
3-Acetyl-2-(benzylsulfonyl)-8-brom-5-chlorchinolin-4(1H)-on,
3-Acetyl-8-brom-5-chlor-2-((2,5-dichlorbenzyl)sulfinyl)chinolin-4(1H)-on,
3-Acetyl-8-brom-5-chlor-2-((3,5-difluorbenzyl)sulfinyl)chinolin-4(1H)-on,
3-Acetyl-8-brom-5-chlor-2-((3-iodbenzyl)sulfinyl)chinolin-4(1H)-on,
3-Acetyl-8-brom-5-chlor-2-((3-fluorbenzyl)sulfinyl)chinolin-4(1H)-on,
3-Acetyl-8-brom-5-chlor-2-(((5-methylisoxazol-3-yl)methyl)sulfinyl)chinolin-4(1H)-on,
1-(2-Benzylsulfinyl)-8-brom-5-chlor-4-hydroxychinolin-3-yl)ethan-1-on,
1-(2-Benzylsulfonyl)-8-brom-5-chlor-4-hydroxychinolin-3-yl)ethan-1-on,
3-Acetyl-8-brom-5-chlor-2-((3-methoxybenzyl)sulfinyl)chinolin-4(1H)-on,
3-Acetyl-8-brom-5-chlor-2-((4-((trifluormethyl)thio)benzyl)sulfinyl)chinolin-4(1H)-on,
3-Acetyl-5,8-dichlor-2-((4-nitrobenzyl)sulfinyl)chinolin-4(1H)-on,
2-(((3-Acetyl-8-brom-5-chlor-4-oxo-1,4-dihydrochinolin-2-yl)sulfinyl)methyl)benzonitril,
3-Acetyl-8-brom-5-chlor-2-((3,5-dimethoxybenzyl)sulfinyl)chinolin-4(1H)-on,
3-Acetyl-8-brom-2-((4-tert-butyl)benzyl)sulfinyl)-5-chlorchinolin-4(1H)-on,
3-Acetyl-2-((4-benzoylbenzyl)sulfinyl)-8-brom-5-chlorchinolin-4(1H)-on,
3-Acetyl-8-brom-5-chlor-2-((4-(trifluormethyl)sulfinyl)benzyl)sulfinyl)chinolin-4(1H)-on,
3-Acetyl-8-brom-5-chlor-2-(4-(pentafluor-16-sulfanyl)benzyl)sulfinyl)chinolin-4(1H)-on,
3-Acetyl-8-brom-5-chlor-2-((2-fluor-4-(pentafluor-16-sulfanyl)benzyl)sulfinyl)chinolin-4(1H)-on,
3-Acetyl-8-brom-5-chlor-2-((4-trifluormethyl)benzyl)sulfinyl)chinolin-4(1H)-on,
3-Acetyl-8-brom-5-chlor-2-((4-trifluormethoxy)benzyl)sulfinyl)chinolin-4(1H)-on,
3-Acetyl-8-brom-5-chlor-2-(((5-(trifluormethyl)furan-2-yl)methyl)sulfinyl)chinolin-4(1H)-on,
4-(((3-Acetyl-8-brom-5-chlor-4-oxo-1,4-dihydrochinolin-2-yl)sulfinyl)methyl)benzonitril,
3-Acetyl-8-brom-5-chlor-2-((2-chlor-6-fluorbenzyl)sulfinyl)chinolin-4(1H)-on,
3-Acetyl-8-brom-5-chlor-2-((2-methoxy-4-(pentafluor-16-sulfanyl)benzyl)sulfinyl)chinolin-4(1H)-on,
3-Acetyl-8-brom-5-chlor-2-((3-fluor-5-(pentafluor-16-sulfanyl)benzyl)sulfinyl)chinolin-4(1H)-on,
3-Acetyl-8-brom-5-chlor-2-((3-(pentafluor-16-sulfanyl)benzyl)sulfinyl)chinolin-4(1H)-on,
3-Acetyl-8-brom-5-chlor-2-(((perfluorphenyl)methyl)sulfinyl)chinolin-4(1H)-on,
3-Acetyl-5,8-dichlor-2-((4-((trifluormethyl)thio)benzyl)sulfinyl)chinolin-4(1H)-on,
3-Acetyl-5,8-difluor-2-((4-(pentafluor-16-sulfanyl)benzyl)sulfinyl)chinolin-4(1H)-on,
3-Acetyl-5,8-difluor-2-(((5-(trifluormethyl)furan-2-yl)methyl)sulfinyl)chinolin-4(1H)-on,
3-Acetyl-5,8-difluor-2-(((5-methylisoxazol-3-yl)methyl)sulfinyl)chinolin-4(1H)-on,
3-Acetyl-5,8-dichlor-2-((4-iodbenzyl)sulfinyl)chinolin-4(1H)-on,
3-Acetyl-8-brom-5-chlor-2-((pyridin-3-ylmethyl)sulfinyl)chinolin-4(1H)-on,
5,8-Difluor-3-isobutyryl-2-((4-((trifluormethyl)thio)benzyl)sulfinyl)chinolin-4(1H)-on,
5,8-Dichlor-3-isobutyryl-2-(((5-methylisoxazol-3-yl)methyl)sulfinyl)chinolin-4(1H)-on,
3-Benzoyl-5,8-difluor-2-((4-pentafluor-16-sulfanyl)benzyl)sulfinyl)chinolin-4(1H)-on,
3-Benzoyl-5,8-dichlor-2-(((5-methylisoxazol-3-yl)methyl)sulfinyl)chinolin-4(1H)-on, Methyl-5-(((3-acetyl-5,8-dichlor-4-oxo-1,4-dihydrochinolin-2-yl)sulfinyl)methyl)furan-2-carboxylat,
Methyl-4-(((3-acetyl-5,8-dichlor-4-oxo-1,4-dihydrochinolin-2-yl)sulfinyl)methyl)benzoat,
3-Acetyl-5-methoxy-2-((4-pentafluor-16-sulfanyl)benzyl)sulfinyl)chinolin-4(1H)-on, 3-Acetyl-5-methoxy-2-(((5-methylisoxazol-3-yl)methyl)sulfinyl)chinolin-4(1H)-on,
8-Brom-5-chlor-2-isobutyryl-2-(((5-methylisoxazol-3-yl)methyl)sulfinyl)chinolin-4(1H)-on,
8-Brom-5-chlor-3-(cyclopropancarbonyl)-2-(((5-methylisoxazol-3-yl)methyl)sulfinyl)chinolin-4(1H)-on,
5,8-Dichlor-3-(cyclopropancarbonyl)-2-(((5-methylisoxazol-3-yl)methyl)sulfinyl)chinolin-4(1H)-on,
5-(((3-Acetyl-8-brom-5-chlor-4-oxo-1,4-dihydrochinolin-2-yl)sulfinyl)methyl)thiophen-2-carbonitril,
2-(((6-(1H-Pyrazol-1-yl)pyridin-3-yl)methyl)sulfinyl)-3-acetyl-8-brom-5-chlorchinolin-4(1H)-on,
3-Acetyl-2-(((6-aminopyridin-3-yl)methyl)sulfinyl)-8-brom-5-chlorchinolin-4(1H)-on,
8-Brom-5-chlor-3-(cyclopropancarbonyl)-2-((4-((trifluormethyl)thio)benzyl)sulfinyl)chinolin-4(1H)-on,
3-Acetyl-8-brom-5-chlor-2-(((2-methyl-6-(trifluormethyl)pyridin-3-yl)methyl)sulfinyl)chinolin-4(1H)-on,
N-(4-(((3-Acetyl-8-brom-5-chlor-4-oxo-1,4-dihydrochinolin-2-yl)sulfinyl)methyl)phenyl)methansulfonamid,
3-Acetyl-8-brom-5-chlor-2-(((6-chlorpyridin-3-yl)methyl)sulfinyl)chinolin-4(1H)-on,
3-Acetyl-8-brom-5-chlor-2-(((6-((2-methoxyethyl)amino)pyridin-3-yl)methyl)sulfinyl)chinolin-4(1H)-on,
3-Acetyl-8-brom-5-chlor-2-(((4-methyl-1,2,5-oxadiazol-3-yl)methyl)sulfinyl)chinolin-4(1H)-on,
2-(((1H-Pyrrolo[2,3-b]pyridin-5-yl)methyl)sulfinyl)-3-acetyl-8-brom-5-chlorchinolin-4(1H)-on,
oder pharmazeutisch akzeptable Salze davon.

4. Zusammensetzung, umfassend Verbindungen nach einem Anspruch der Ansprüche 1 bis 3 oder pharmazeutisch akzeptable Salze davon und einen pharmazeutisch akzeptablen Träger.

5. Verbindung nach einem der Ansprüche 1―3 oder Zusammensetzung nach Anspruch 4 zur Verwendung bei einem Verfahren zur Behandlung oder Prävention von Krebs.

## Revendications

1. Composé de formule 3a ou 3b ou sels pharmaceutiquement acceptables de celui-ci : 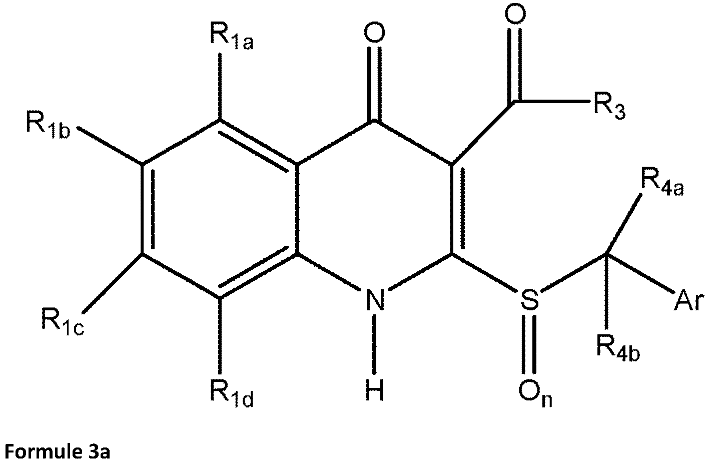 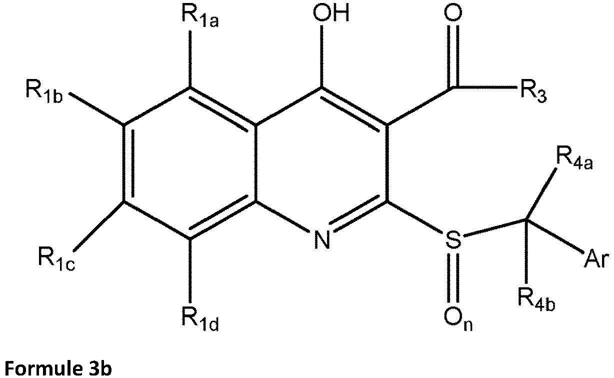 dans laquelle
R₁ₐ à R_{1d} représentent chacun indépendamment un hydrogène, un halogène, un alkyle en C_{1 à 6}, un haloalkyle en C_{1 à 6}, un hydroxyalkyle en C_{1 à 6}, un alcoxy en C_{1 à 6}, un haloalcoxy en C_{1 à 6}, un alcényle en C_{2 à 10}, un haloalcényle en C_{2 à 10}, un alcynyle en C_{2 à 10}, un haloalcynyle en C_{2 à 10}, un groupe hydroxyle, un nitro, un cyano, un alcoxycarbonyle en C_{1 à 6}, un amino, un alkylamino en C_{1 à 6}, un di(alkyle en C_{1 à 6})amino, un aminoalkyle(C_{1 à 6}), un alkylamino(C_{1 à 6})alkyle(C_{1 à 6}), un alcanoyle en C_{1 à 6}, un cycloalkyle en C_{3 à 7}, un aryle, un hétérocycle ou un hétéroaryle, dans laquelle R₁ₐ à R_{1d} sont chacun indépendamment non substitué ou substitué facultativement ;
R₃ représente un alkyle en C_{1 à 4}, un isoalkyle, un cycloalkyle, un phényle ou un haloalkyle en C_{1 à 4} ;
n vaut 1 ou 2 ;
R₄ₐ et R_{4b} représentent chacun indépendamment un hydrogène, un halogène, un alkyle en C_{1 à 4}, un haloalkyle en C_{1 à 4} ou un alkyle en C_{1 à 4} dans lesquels un ou plusieurs hydrogènes sont substitués par des substituants différents de l'halogène ;
Ar représente un phényle, un hétéroaryle présentant de 5 à 6 chaînons et présentant des hétéroatomes choisis indépendamment parmi N, S ou O, ou un bihétéroaryle présentant de 8 à 12 chaînons et présentant des hétéroatomes choisis indépendamment parmi N, S ou O,
dans laquelle Ar peut être non substitué ou peut être substitué facultativement par un ou plusieurs éléments parmi un halogène, un alkyle en C_{1 à 6}, un alkylthio en C_{1 à 6}, un haloalkyle en C_{1 à 6}, un haloalkylthio en C_{1 à 6}, un alcoxy en C_{1 à 6}, un haloalcoxy en C_{1 à 6}, un alcényle en C_{2 à 10}, un haloalcényle en C_{2 à 10}, un alcynyle en C_{2 à 10}, un haloalcynyle en C_{2 à 10}, un groupe hydroxyle, un COOH, un nitro, un cyano, un alcoxycarbonyle en C_{1 à 6}, un amino, un alkylamino en C_{1 à 6}, un di(alkyle en C_{1 à 6})amino, un aminoalkyle(C_{1 à 6}), un alkylamino(C_{1 à 6})alkyle(C_{1 à 6}), un alcoxy(C_{1 à 6})alkylamino(C_{1 à 6}), un alkylamino(C_{1 à 6})alkylamino(C_{1 à 6}), un alkanoyle en C_{1 à 6}, SF5, S(O)CF₃, SCF₃, NHC(=O)CH₃, C(=O)NHCH₃, NHSO2CH3, un cycloalkyle en C_{3 à 7}, un aryle, un benzoyle, un hétérocycle, un hétéroaryle, un phényle, un oxazole, un pyrazole, un pyrrole, un imidazole, un thiazole, un thiophène, un pyridine, un pyrimidine, un furane, un indole, un benzopyrazole, un benzothiazole, un benzooxazole, un isoxazole, un benzoimidazole ou un benzothiophène, et
dans laquelle les substituants de Ar peuvent être non substitués ou peuvent être substitués facultativement par un ou plusieurs CF₃, un halogène, un alkyle en C_{1 à 3}, un haloalkyle en C_{1 à 3}, un hydrogène, un COOH, un nitro, un cyano, un amino, un di(alkyle en C_{1 à 3})amino, un NHC(=O)CH₃ ou un C(=O)NHCH₃.

2. Composés selon la revendication 1, dans lesquels l'un quelconque ou plusieurs parmi R₁ₐ à R_{1d} représentent des halogènes, R₃ représente un alkyle en C_{1 à 4}, un isoalkyle, un cycloalkyle, un phényle ou un haloalkyle en C_{1 à 4} et R₄ₐ et R_{4b} représentent un alkyle en C_{1 à 4}, un halogène ou un cycloalkyle en C_{1 à 4}.

3. Composé selon la revendication 1 choisi parmi le groupe consistant en :
3-acétyl-2(benzylsulfinyl)-8-bromo-5-chloroquinolin-4(1H)-one,
3-acétyl-8-bromo-5-chloro-2-((4-chlorobenzyl)sulfinyl)quinolin-4(1H)-one,
3-acétyl-8-bromo-5-chloro-2-(phénylsulfinyl)quinolin-4(1H)-one,
3-acétyl-8-bromo-5-chloro-2-((2-méthoxyphényl)sulfinyl)quinolin-4(1H)-one,
3-acétyl-8-bromo-2-((4-bromophényl)sulfinyl)-5-chloroquinolin-4(1H)-one,
3-acétyl-8-bromo-5-chloro-2-((1-phényléthyl)sulfinyl)quinolin-4(1H)-one,
3-(((3-acétyl-8-bromo-5-chloro-4-oxo-1,4-dihydroquinolin-2-yl)sulfinyl)méthyl)benzonitrile,
3-acétyl-8-bromo-5-chloro-2-((2,4-difluorobenzyll)sulfinyl)quinolin-4(1H)-one,
3-acétyl-8-bromo-5-chloro-2-((3-chloro-4-fluorobenzyl)sulfinyl)quinolin-4(1H)-one,
3-acétyl-8-bromo-5-chloro-2-((4-nitrobenzyl)sulfinyl)quinolin-4(1H)-one,
3-acétyl-2-(benzylsulfonyl)-8-bromo-5-chloroquinolin-4(1H)-one,
3-acétyl-8-bromo-5-chloro-2-((2,5-dichlorobenzyl)sulfinyl)quinolin-4(1H)-one,
3-acétyl-8-bromo-5-chloro-2-((3,5-difluorobenzyl)sulfinyl)quinolin-4(1H)-one,
3-acétyl-8-bromo-5-chloro-2-((3-iodobenzyl)sulfinyl)quinolin-4(1H)-one,
3-acétyl-8-bromo-5-chloro-2-((3-fluorobenzyl)sulfinyl)quinolin-4(1H)-one,
3-acétyl-8-bromo-5-chloro-2-(((5-méthylisoxazol-3-yl)méthyl)sulfinyl)quinolin-4(1H)-one,
1-(2-benzylsulfinyl)-8-bromo-5-chloro-4-hydroxyquinolin-3-yl)éthan-1-one,
1-(2-benzylsulfonyl)-8-bromo-5-chloro-4-hydroxyquinolin-3-yl)éthan-1-one,
3-acétyl-8-bromo-5-chloro-2-((3-méthoxybenzyl)sulfinyl)quinolin-4(1H)-one,
3-acétyl-8-bromo-5-chloro-2-((4-((trifluorométhyl)thio)benzyl)sulfinyl)quinolin-4(1H)-one,
3-acétyl-5,8-dichloro-2-((4-((nitrobenzyl)sulfinyl)quinolin-4(1H)-one,
2-(((3-acétyl-8-bromo-5-chloro-4-oxo-1,4-dihydroquinolin-2-yl)sulfinyl)méthyl)benzonitrile,
3-acétyl-8-bromo-5-chloro-2-((3,5-diméthoxybenzyl)sulfinyl)quinolin-4(1H)-one,
3-acétyl-8-bromo-2-((4-tert-butyl)benzyl)sulfinyl)-5-chloroquinolin-4(1H)-one,
3-acétyl-2-((4-benzoylbenzyl)sulfinyl)-8-bromo-5-chloroquinolin-4(1H)-one,
3-acétyl-8-bromo-5-chloro-2-((4-((trifluorométhyl)sulfinyl)benzyl)sulfinyl)quinolin-4(1H)-one,
3-acétyl-8-bromo-5-chloro-2-((4-((pentafluoro-16-sulfanyl)benzyl)sulfinyl)quinolin-4(1H)-one,
3-acétyl-8-bromo-5-chloro-2-((2-fluoro-4-(pentafluoro-16-sulfanyl)benzyl)sulfinyl)quinolin-4(1H)-one,
3-acétyl-8-bromo-5-chloro-2-((4-(trifluorométhyl)benzyl)sulfinyl)quinolin-4(1H)-one,
3-acétyl-8-bromo-5-chloro-2-((4-(trifluorométhoxy)benzyl)sulfinyl)quinolin-4(1H)-one,
3-acétyl-8-bromo-5-chloro-2-(((5-(trifluorométhyl)furan-2-yl)méthyl)sulfinyl)quinolin-4(1H)-one,
4-(((3-acétyl-8-bromo-5-chloro-4-oxo-1,4-dihydroquinolin-2-yl)sulfinyl)méthyl)benzonitrile,
3-acétyl-8-bromo-5-chloro-2-((2-chloro-6-fluorobenzyl)sulfinyl)quinolin-4(1H)-one,
3-acétyl-8-bromo-5-chloro-2-((2-méthoxy-4-(pentafluoro-16-sulfanyl)benzyl)sulfinyl)quinolin-4(1H)-one,
3-acétyl-8-bromo-5-chloro-2-((3-fluoro-5-(pentafluoro-16-sulfanyl)benzyl)sulfinyl)quinolin-4(1H)-one,
3-acétyl-8-bromo-5-chloro-2-((3-(pentafluoro-16-sulfanyl)benzyl)sulfinyl)quinolin-4(1H)-one,
3-acétyl-8-bromo-5-chloro-2-(((perfluorophényl)méthyl)sulfinyl)quinolin-4(1H)-one,
3-acétyl-5,8-dichloro-2-((4-((trifluorométhyl)thio)benzyl)sulfinyl)quinolin-4(1H)-one,
3-acétyl-5,8-difluoro-2-((4-((pentafluoro-16-sulfanyl)benzyl)sulfinyl)quinolin-4(1H)-one,
3-acétyl-5,8-difluoro-2-(((5-(trifluorométhyl)furan-2-yl)méthyl)sulfinyl)quinolin-4(1H)-one,
3-acétyl-5,8-difluoro-2-(((5-méthylisoxazol-3-yl)méthyl)sulfinyl)quinolin-4(1H)-one,
3-acétyl-5,8-dichloro-2-((4-iodobenzyl)sulfinyl)quinolin-4(1H)-one,
3-acétyl-8-bromo-5-chloro-2-((pyridin-3-ylméthyl)sulfinyl)quinolin-4(1H)-one,
5,8-difluoro-3-isobutyryl-2-((4-((trifluorométhyl)thio)benzyl)sulfinyl)quinolin-4-(1H)-one,
5,8-dichloro-3-isobutyryl-2-(((5-méthylisoxazol-3-yl)méthyl)sulfinyl)quinolin-4-(1H)-one,
3-benzoyl-5,8-difluoro-2-((4-(pentafluoro-16-sulfanyl)benzyl)sulfinyl)quinolin-4-(1H)-one,
3-benzoyl-5,8-dichloro-2-(((5-méthylisoxazol-3-yl)méthyl)sulfinyl)quinolin-4-(1H)-one,
méthyle 5-(((3-acétyl-5,8-dichloro-4-oxo-1,4-dihydroquinolin-2-yl)sulfinyl)méthyl)furan-2-carboxylate,
méthyle 4-(((3-acétyl-5,8-dichloro-4-oxo-1,4-dihydroquinolin-2-yl)sulfinyl)méthyl)benzoate,
3-acétyl-5-méthoxy-2-((4-pentafluoro-16-sulfanyl)benzyl)sulfinyl)quinolin-4(1H)-one,
3-acétyl-5-méthoxy-2-(((5-méthylisoxazol-3-yl)méthyl)sulfinyl)quinolin-4(1H)-one,
8-bromo-5-chloro-3-isobutyryl-2-(((5-méthylisoxazol-3-yl)méthyl)sulfinyl)quinolin-4-(1H)-one,
8-bromo-5-chloro-3-(cyclopropanecarbonyl)-2-(((5-méthylisoxazol-3-yl)méthyl)sulfinyl)quinolin-4-(1H)-one,
5,8-dichloro-3-(cyclopropanecarbonyl)-2-(((5-méthylisoxazol-3-yl)méthyl)sulfinyl)quinolin-4-(1H)-one,
5-(((3-acétyl-8-bromo-5-chloro-4-oxo-1,4-dihydroquinolin-2-yl)sulfinyl)méthyl)thiophène)-2-carbonitrile,
2-(((6-(1H-pyrazol-1-yl)pyridin-3-yl)méthyl)sulfinyl)-3-acétyl-8-bromo-5-chloroquinolin-4(1H)-one,
3-acétyl-2-(((6-aminopyridin-3-yl)méthyl)sulfinyl)-8-bromo-5-chloroquinolin-4(1H)-one,
8-bromo-5-chloro-3-(cyclopropanecarbonyl)-2-((4-((trifluorométhyl)thio)benzyl)sulfinyl)quinolin-4(1H)-one,
3-acétyl-8-bromo-5-chloro-2-(((2-méthyl-6-trifluorométhyl)pyridin-3-yl)méthyl)sulfinyl)quinolin-4(1H)-one,
N-(4-(((3-acétyl-8-bromo-5-chloro-4-oxo-1,4-dihydroquinolin-2-yl)sulfinyl)méthyl)phényl)méthanesulfonamide,
3-acétyl-8-bromo-5-chloro-2-(((6-chloropyridin-3-yl)méthyl)sulfinyl)quinolin-4(1H)-one,
3-acétyl-8-bromo-5-chloro-2-(((6-((2-méthoxyéthyl)amino)pyridin-3-yl)méthyl)sulfinyl)quinolin-4(1H)-one,
3-acétyl-8-bromo-5-chloro-2-(((4-méthyl-1,2,5-oxadiazol-3-yl)méthyl)sulfinyl)quinolin-4(1H)-one,
2-(((1H-pyrrolo[2,3-b]pyridin-5-yl)méthyl)sulfinyl)-3-acétyl-8-bromo-5-chloroquinolin-4(1H)-one,
ou des sels pharmaceutiquement acceptables de ceux-ci.

4. Composition comprenant des composés selon l'une quelconque des revendications 1 à 3 ou des sels pharmaceutiquement acceptables de celle-ci, et un vecteur pharmaceutiquement acceptable.

5. Composé selon l'une quelconque des revendications 1-3, ou composition selon la revendication 4, destiné(e) à être utilisé(e) dans un procédé de traitement ou de prévention d'un cancer.
